# EUROPÄISCHE PATENTSCHRIFT

(11) **EP 2 041 125 B1**
(45) Veröffentlichungstag und Bekanntmachung des Hinweises auf die Patenterteilung: **26.01.2011**
(21) Anmeldenummer: 07765594.2
(22) Anmeldetag: 25.06.2007
(51) Int. Cl.: C07D 409/14, C07D 405/14, C07D 413/14, C07D 417/14, A01N 43/653

(54) **AZOLYLMETHYLOXIRANE, IHRE VERWENDUNG ZUR BEKÄMPFUNG VON PFLANZENPATHOGENEN PILZEN SOWIE SIE ENTHALTENDE MITTEL**
AZOLYLMETHYLOXIRANES, THEIR USE FOR CONTROLLING PHYTOPATHOGENIC FUNGI AND AGENTS CONTAINING SAID COMPOUNDS
AZOLYLMÉTHYLOXYRANES, LEUR UTILISATION POUR LUTTER CONTRE DES CHAMPIGNONS PHYTOPATHOGÈNES ET AGENTS LES CONTENANT

(30) Priorität: 05.07.2006 EP 06116611
(43) Veröffentlichungstag der Anmeldung: 01.04.2009
(73) Patentinhaber: BASF SE, 67056 Ludwigshafen (DE)
(72) Erfinder: DIETZ, Jochen, 68167 Mannheim (DE); GROTE, Thomas, 67157 Wachenheim (DE); MÜLLER, Bernd, 67227 Frankenthal (DE); LOHMANN, Jan Klaas, 67063 Ludwigshafen (DE); RENNER, Jens, 67098 Bad Dürkheim (DE); ULMSCHNEIDER, Sarah, 67098 Bad Dürkheim (DE); GLÄTTLI, Alice, 68165 Mannheim (DE)
(86) Internationale Anmeldenummer: PCT/EP2007/056306
(87) Internationale Veröffentlichungsnummer: WO 2008/003607

(56) Entgegenhaltungen:
- EP-A- 0 421 125
- DE-A1- 3 942 333

## Beschreibung

Die vorliegende Erfindung betrifft Azolylmethyloxirane der allgemeinen Formel I 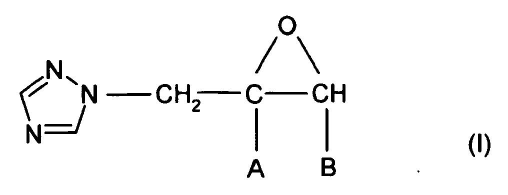 worin
- A oder B: für Thienyl steht, das durch einen bis drei der folgenden Substituenten Ha- logen, C₁-C₄-Alkyl, C₁-C₄-Alkoxy, C₁-C₄-Halogenalkyl oder C₁-C₄- Halogenalkoxy substituiert ist,
und der jeweils andere Substituent
- A oder B: für Phenyl steht, das durch ein bis drei der folgenden Substituenten Halo- gen, C₁-C₄-Alkyl, C₁-C₄-Alkoxy, C₁-C₄-Halogenalkyl oder C₁-C₄- Halogenalkoxy substituiert ist,
sowie deren für Pflanzen verträgliche Säureadditions- oder Metallsalze.

Weiterhin betrifft die Erfindung die Verwendung der Verbindungen der Formel I zur Bekämpfung von pflanzenpathogenen Pilzen und sie enthaltende Mittel.

Azolylmethyloxirane, ihre Herstellung und ihre Verwendung im Pflanzenschutz sind beispielsweise aus der EP-A 0 094 564 und der EP-A 0 196 038 bekannt.

Aus der EP-A 0 421 125 sind Azolylmethyloxirane bekannt, die am Oxiran-Ring einen Hetaryl-Substituenten tragen.

Obwohl die beschriebenen Azolylmethyloxirane bereits eine gute bis sehr gute fungizide Wirkung gegen eine Reihe von Pathogenen aufweisen, lag der vorliegenden Erfindung als Aufgabe zugrunde, neue Azolylmethyloxirane mit einer verbesserten fungiziden Wirkung zur Verfügung zu stellen.

Diese Aufgabe wurde mit den eingangs beschriebenen Verbindungen der Formel I gelöst.

Die Verbindung I ist wegen des basischen Charakters der in ihnen enthaltenen Stickstoffatome in der Lage, mit anorganischen oder organischen Säuren oder mit Metallionen Salze oder Addukte zu bilden.

Beispiele für anorganische Säuren sind Halogenwasserstoffsäuren wie Fluorwasserstoff, Chlorwasserstoff, Bromwasserstoff und Jodwasserstoff, Kohlensäure, Schwefelsäure, Phosphorsäure und Salpetersäure.

Als organischen Säuren kommen beispielsweise Ameisensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxalsäure, Alkylsulfonsäuren (Sulfonsäuren mit geradkettigen oder verzweigten Alkylresten mit 1 bis 20 Kohlenstoffatomen), Arylsulfonsäuren oder -disulfonsäuren (aromatische Reste wie Phenyl und Naphthyl welche eine oder zwei Sulfonsäuregruppen tragen), Alkylphosphonsäuren (Phosphonsäuren mit geradkettigen oder verzweigten Alkylresten mit 1 bis 20 Kohlenstoffatomen), Arylphosphonsäuren oder - diphosphonsäuren (aromatische Reste wie Phenyl und Naphthyl welche eine oder zwei Phosphorsäurereste tragen), wobei die Alkyl- bzw. Arylreste weitere Substituenten tragen können, z.B. p-Toluolsulfonsäure, Salizylsäure, p-Aminosalizylsäure, 2-Phenoxybenzoesäure, 2-Acetoxybenzoesäure etc.

Als Metallionen kommen insbesondere die lonen der Elemente der zweiten Hauptgruppe, insbesondere Calzium und Magnesium, der dritten und vierten Hauptgruppe, insbesondere Aluminium, Zinn und Blei, sowie der ersten bis achten Nebengruppe, insbesondere Chrom, Mangan, Eisen, Kobalt, Nickel, Kupfer, Zink und andere in Betracht. Besonders bevorzugt sind die Metallionen der Elemente der Nebengruppen der vierten Periode. Die Metalle können dabei in den verschiedenen ihnen zukommenden Wertigkeiten vorliegen.

Die Herstellung der Verbindungen der Formel I ist bekannt und in den EP-A 0 094 564, EP-A 0 196 038 und EP-A 0 421 125 ausführlich beschrieben.

Ein wesentliches Intermediat für die Synthese der Zielverbindungen sind α,β-disubstituierte Acroleine vom Typ.

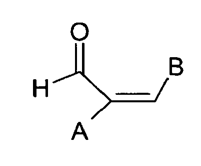

Die Synthese dieser Verbindungen ist u.a. beschrieben in DE3601927 durch Reaktion von Verbindungen des Typs 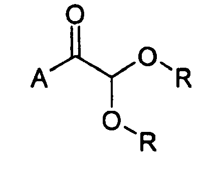 mit Phosphorverbindungen vom Wittig oder Horner-Emmons-Typ mit anschliessender saurer Spaltung des Acetals. R steht bevorzugt für C₁-C₄-Alkyl.

Eine Alternative hierzu besteht in der Synthese von α,β-disubstituierten Acrylsäureestern des Typs:

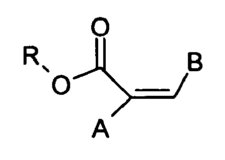

Die Reduktion zum Acrolein kann z.B. mit Metallhydriden wie z.B. Diisobutylaluminiumhydrid bei tiefen Temperaturen durchgeführt werden. Eine weitere Möglichkeit besteht darin, den Ester zum entsprechenden Alkohol zu reduzieren, um in einer anschließenden Oxidation die gewünschte Oxidationsstufe zu erhalten.

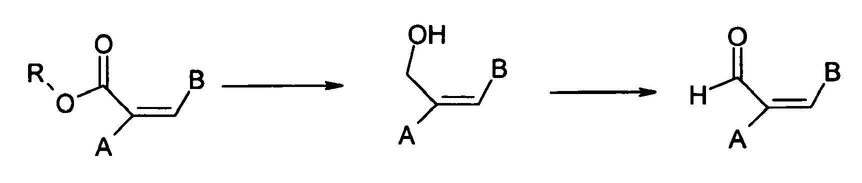

Insbesondere Aluminiumhydriden, bevorzugt Lithiumalanat (European Journal of Medicinal Chemistry, 40(6), 529-541; 2005) oder Dialkylaluminiumhydriden wie zB DIBAL-H (Synlett, (18), 3182-3184; 2006) sind hierbei einsetzbar.

Die entsprechenden Oxidationsreaktionen sind dem Fachmann bekannt, wie z.B. Umsetzungen nach Swern (Australian Journal of Chemistry, 57(6), 537-548; 2004), mit hypervalenten lodverbindungen (Organic Letters, 5(17), 2989-2992; 2003), mit Chromverbindungen wie z.B. Pyridiniumdichromat (Tetrahedron, 45(1), 239-58; 1989) oder mit Manganoxiden, wie z.B. MnO2 (Journal of the American Chemical Society, 107(13), 3963-71; 1985).

Die Acrylsäureester sind zugänglich aus Glyoxalsyäureestern durch Reaktion mit Phosporverbindungen, z.B. vom Horner-Emmons-Typ oder Wittig-Verbindungen. 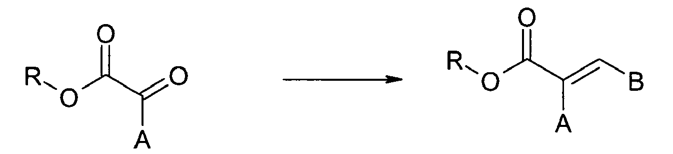 Solche Umsetzungen sind beschrieben u.a. in Tetrahedron, 46(13-14), 4951-94; 1990, Tetrahedron Letters, 47(16), 2675-2678; 2006, Synthesis, (12), 1797-1802; 2003, WO9929645 oder Synthetic Communications, 20(12), 1781-91; 1990.

Die Synthese von Glyoxalsäureestern ist beschrieben u.a. in Journal of Organic Chemistry, 52(22), 5026-30; 1987 durch Umsetzung von Grignard-Verbindungen (ausgehend von kommerziell erhältlichen Halogenverbindungen und Magnesium) mit Oxalsäureestern.

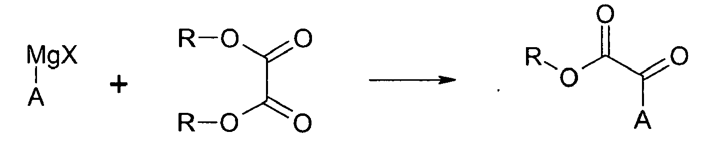

Geeignete Phosphorverbindungen (Horner-Emmons-Typ und Wittig-Typ) sind darstellbar nach bekannten Standardmethoden, z.B. aus einer Verbindung des folgenden Typs:

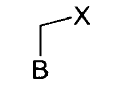

B ist wie oben definiert, X steht für eine Abgangsgruppe wie z.B. ein Halogenid, bevorzugt Chlor oder Brom, ganz besonders Chlor. Die Umsetzung solcher Halogenide zu den gewünschten Horner-Emmons oder Wittig-Reagenzien kann erfolgen wie z.B. in DE2651968.

Die Alkylhalogenide sind entweder käuflich oder darstellbar nach Standardmethoden, z.B. durch Chlorierung des entsprechenden Alkohols mit einem Chlorierungsmittel wie z.B. Thionylchlorid (Tetrahedron: Asymmetry, 14(21), 3281-3283; 2003) oder Salzsäure (Acta Chemica Scandinavica, 51 (3, Suppl.), 392-402; 1997).

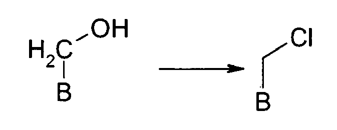

Die Darstellung entsprechender Bromide ist z.B. beschrieben in Bioorganic & Medicinal Chemistry Letters, 16(1), 225-227; 2006 durch Umsetzung der Methylverbindung mit einem Bromierungsmittel, geeigneterweise N-Bromsuccinimid.

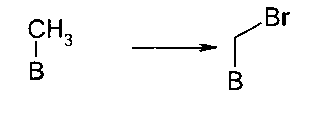

Bei den in den vorstehenden Formeln angegebenen Definitionen der Symbole wurden Sammelbegriffe verwendet, die allgemein repräsentativ für die folgenden Substituenten stehen:
Halogen: Fluor, Chlor, Brom und Jod;
Alkyl sowie die Alkylteile von zusammengesetzten Gruppen wie beispielsweise Alkylamino: gesättigte, geradkettige oder verzweigte Kohlenwasserstoffreste mit vorzugsweise 1 bis 4 Kohlenstoffatomen, wie Methyl, Ethyl, Propyl, 1-Methylethyl, Butyl, 1-Methyl-propyl, 2-Methylpropyl und 1,1-Dimethylethyl.
Halogenalkyl: Alkyl wie vorstehend genannt, wobei in diesen Gruppen teilweise oder vollständig die Wasserstoffatome durch Halogenatome wie vorstehend genannt ersetzt sind. In einer Ausführungsform sind die Alkylgruppen mindestens ein Mal oder vollständig durch ein bestimmtes Halogenatom substituiert, vorzugsweise Fluor, Chlor oder Brom. In einer weiteren Ausführungsform sind die Alkylgruppen durch verschiedene Halogenatome teilweise oder vollständig halogeniert; bei gemischten Halogensubstitutionen ist die Kombination Chlor und Fluor bevorzugt. Insbesondere bevorzugt sind (C₁-C₄)-Halogenalkyl, mehr bevorzugt (C₁-C₂)-Halogenalkyl, wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl oder 1,1,1-Trifluorprop-2-yl;
Alkoxy: für eine über ein Sauerstoff gebundene Alkylgruppe wie oben definiert, bevorzugt mit 1 bis 4 C-Atomen. Beispiele für bevorzugte Alkoxygruppen sind: Methoxy, E-thoxy, n-Propoxy, 1-Methylethoxy, Butoxy, 1-Methylpropoxy, 2-Methylpropoxy oder 1,1-Dimethylethoxy.
Halogenalkoxy: Alkoxy, wie vorstehend definiert, wobei in diesen Gruppen die Wasserstoffatome teilweise oder vollständig gegen Halogenatome, wie vorstehend unter Halogenalkyl beschrieben, insbesondere Fluor, Chlor oder Brom, ersetzt sind. Beispiele für bevorzugte Halogenalkoxyreste sind OCH₂F, OCHF₂, OCF₃, OCH₂Cl, OCHCl₂, OCCl₃, Chlorfluormethoxy, Dichlorfluormethoxy, Chlordifluormethoxy, 2-Fluorethoxy, 2-Chlorethoxy, 2-Bromethoxy, 2-lodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, OC₂F₅, 2-Fluorpropoxy, 3-Fluorpropoxy, 2,2-Difluorpropoxy, 2,3-Difluorpropoxy, 2-Chlorpropoxy, 3-Chlorpropoxy, 2,3-Dichlorpropoxy, 2-Brompropoxy, 3-Brompropoxy, 3,3,3-Trifluorpropoxy, 3,3,3-Trichlorpropoxy, OCH₂-C₂F₅, OCF₂-C₂F₅, 1-(CH₂F)-2-fluorethoxy, 1-(CH₂Cl)-2-chlorethoxy, 1-(CH₂Br)-2-bromethoxy, 4-Fluorbutoxy, 4-Chlorbutoxy, 4-Brombutoxy oder Nonafluorbutoxy.
Alkylthio: Alkyl, wie vorstehend definiert, das über ein S-Atom gebunden ist.
5-gliedriges Heteroaryl: Furyl, Thienyl, Pyrrolyl, Pyrazolyl, Imidazolyl, Oxazolyl, Isoxazolyl, Thiazolyl und Isothiazolyl, insbesondere 2-Furyl, 3-Furyl, 2-Thienyl, 3-Thienyl, 2-Pyrrolyl, 3-Pyrrolyl, 3-Isoxazolyl, 4-lsoxazolyl, 5-lsoxazolyl, 3-lsothiazolyl, 4- Isothiazolyl, 5- Isothiazolyl, 3-Pyrazolyl, 4- Pyrazolyl, 5- Pyrazolyl, 2-Oxazolyl, 3-Oxazolyl, 4-Oxyzolyl, 5-Oxazolyl, 2-Thiazolyl, 4-Thiazolyl, 5-Thiazolyl, 2-lmidazolyl und 4-Imidazolyl. Das Heteroaryl kann über C oder N, falls vorhanden, angebunden sein.

Die neuen Verbindungen der Formel I enthalten chirale Zentren und werden im allgemeinen in Form von Racematen oder als Diastereomerengemische von erythro- sowie threo-Formen erhalten. Die erythro- und threo-Diastereomeren lassen sich bei den erfindungsgemäßen Verbindungen beispielsweise aufgrund ihrer unterschiedlichen Löslichkeit oder durch Säulenchromatographie trennen und in reiner Form isolieren. Aus solchen einheitlichen Diastereomerenpaaren kann man mit bekannten Methoden einheitliche Enantiomere erhalten. Als antimikrobielle Mittel kann man sowohl die einheitlichen Diastereomere bzw. Enantiomere wie auch deren bei der Synthese anfallende Gemische verwenden. Entsprechendes gilt für die fungiziden Mittel.

Die erfindungsgemäßen Verbindungen können in verschiedenen Kristallmodifikationen vorliegen, die sich in der biologischen Wirksamkeit unterscheiden können. Sie sind ebenfalls Gegenstand der vorliegenden Erfindung.

In den erfindungsgemäßen bzw. erfindungsgemäß verwendeten Verbindungen der Formel I sind die folgenden Bedeutungen der Substituenten, und zwar jeweils für sich allein oder in Kombination, besonders bevorzugt. Die bevorzugten Substituenten oder bevorzugten Kombinationen von Substituenten gelten dabei ggf. entsprechend für die Vorstufen der erfindungsgemäßen Verbindungen.

Der Substituent A oder B steht für Thienyl, das durch einen bis drei der folgenden Substituenten Halogen, C₁-C₄-Alkyl, C₁-C₄-Alkoxy, C₁-C₄-Halogenalkyl oder C₁-C₄-Halogenalkoxy substituiert ist.

Gemäß einer anderen Ausführungsform steht der Substituent A oder B für Thienyl, das durch ein bis drei der o.g. Substituenten substituiert ist.

Gemäß einer weiteren Ausführungsform ist das Thienyl durch ein bis drei der folgenden Substituenten Halogen, C₁-C₄-Alkyl, C₁-C₄-Alkoxy, C₁-C₄-Halogenalkyl oder C₁-C₄-Halogenalkoxy, substituiert.

Gemäß einer anderen Ausführungsform ist das Thienyl durch ein bis drei der folgenden Substituenten Halogen, C₁-C₄-Alkyl oder C₁-C₄-Alkoxy substituiert.

Gemäß einer bevorzugten Ausführungsform ist das Thienyl durch ein bis drei der folgenden Substituenten F, Cl, Methyl oder Methoxy substituiert.

Gemäß einer weiteren bevorzugten Ausführungsform ist das Thienyl durch ein bis drei der folgenden Substituenten F oder Cl substituiert.

Gemäß einer Ausführungsform steht der jeweils andere vom Thienyl unterschiedliche Substituent A oder B für Phenyl, das durch ein bis drei der folgenden Substituenten Halogen, C₁-C₄-Alkyl, C₁-C₄-Alkoxy, C₁-C₄-Halogenalkyl oder C₁-C₄-Halogenalkoxy, substituiert ist.

Gemäß einer weiteren Ausführungsform steht der jeweils andere vom Thienyl unterschiedliche Substituent A oder B für Phenyl, das durch ein bis drei der folgenden Substituenten Halogen, C₁-C₄-Alkyl oder C₁-C₄-Alkoxy, substituiert ist.

Gemäß einer weiteren Ausführungsform steht der jeweils andere vom Thienyl unterschiedliche Substituent A oder B für Phenyl, das durch ein bis drei der folgenden Substituenten Halogen, substituiert ist.

Gemäß einer weiteren Ausführungsform steht der jeweils andere vom Thienyl unterschiedliche Substituent A oder B für Phenyl, das durch ein bis drei der folgenden Substituenten F, Cl, Methyl oder Methoxy, substituiert ist.

Gemäß einer weiteren Ausführungsform steht der jeweils andere vom Thienyl unterschiedliche Substituent A oder B für Phenyl, das durch ein bis drei der folgenden Substituenten F oder Cl, substituiert ist.

In einer bevorzugten Ausführungsform steht B für Phenyl, das durch ein bis drei Halogen substituiert ist.

Insbesondere sind im Hinblick auf ihre Verwendung die in den folgenden Tabellen 5 bis 70 zusammengestellten erfindungsgemäßen Verbindungen I bevorzugt. Die in den Tabellen für einen Substituenten genannten Gruppen stellen außerdem für sich betrachtet, unabhängig von der Kombination, in der sie genannt sind, eine besonders bevorzugte Ausgestaltung des betreffenden Substituenten dar.

Mit [*] gekennzeichnete Zeilen in der Tabelle sind nicht vom Schutzumfang umfasst.

**Tabelle 1**

| Zeile | Substituent A |
|---|---|
| 1-1 | 2-Chlor-thien-3-yl |
| 1-2 | 4-Chlor-thien-3-yl |
| 1-3 | 5-Chlor-thien-3-yl |
| 1-4 | 2,5-Dichlor-thien-3-yl |
| 1-5 | 2,4,5-Trichlor-thien-3-yl |
| 1-6 | 2-Brom-thien-3-yl |
| 1-7 | 4-Brom-thien-3-yl |
| 1-8 | 5-Brom-thien-3-yl |
| 1-9 | 2,5-Dibrom-thien-3-yl |
| 1-10 | 2,4,5-Tribrom-thien-3-yl |
| 1-11 | 3-Brom-fur-2-yl [*] |
| 1-12 | 4-Brom-fur-2-yl [*] |
| 1-13 | 5-Brom-fur-2-yl [*] |
| 1-14 | 4,5-Dibrom-fur-2-yl [*] |
| 1-15 | 4-Chlor-fur-2-yl [*] |
| 1-16 | 5-Chlor-fur-2-yl [*] |
| 1-17 | 3,4-Dichlor-fur-2-yl [*] |
| 1-18 | 2-Chlor-thiazol-5-yl [*] |
| 1-19 | 4-Chlor-thiazol-5-yl [*] |
| 1-20 | 2,4-Dichlor-thiazol-5-yl [*] |
| 1-21 | 2-Brom-thiazol-5-yl [*] |
| 1-22 | 2-Chlor-thiazol-4-yl [*] |
| 1-23 | 2-Brom-thiazol-4-yl [*] |
| 1-24 | 1-Methyl-pyrazol-4-yl [*] |
| 1-25 | 1,3-Dimethyl-pyrazol-4-yl [*] |
| 1-26 | 1,5-Dimethyl-pyrazol-4-yl [*] |
| 1-27 | 1,3,5-Trimethylpyrazol-4-yl [*] |
| 1-28 | 1-Methyl-pyrazol-3-yl [*] |
| 1-29 | 1-Methyl-imidazol-4-yl [*] |
| 1-30 | 1,5-Dimethyl-imidazol-4-yl [*] |
| 1-31 | 1-Methyl-pyrazol-5-yl [*] |
| 1-32 | 1,2-Dimethyl-imidazol-5-yl [*] |
| 1-33 | 1,4-Dimethyl-imidazol-5-yl [*] |

**Tabelle 2**

| Zeile | Substituent B |
|---|---|
| 2-1 | Phenyl [*] |
| 2-2 | 2-Methylphenyl |
| 2-3 | 3-Methylphenyl |
| 2-4 | 4-Methylphenyl |
| 2-5 | 2-Methoxyphenyl |
| 2-6 | 3-Methoxyphenyl |
| 2-7 | 4-Methoxyphenyl |
| 2-8 | 2-Chlorphenyl |
| 2-9 | 3-Chlorphenyl |
| 2-10 | 4-Chlorphenyl |
| 2-11 | 2-Fluorphenyl |
| 2-12 | 3-Fluorphenyl |
| 2-13 | 4-Fluorphenyl |
| 2-14 | 2-Chlor-3-Methoxyphenyl |
| 2-15 | 2-Chlor-4-Methoxyphenyl |
| 2-16 | 2,3-Dichlorphenyl |
| 2-17 | 2,4-Dichlorphenyl |
| 2-18 | 3,4-Dichlorphenyl |
| 2-19 | 2,3-Difluorphenyl |
| 2-20 | 2,4-Difluorphenyl |
| 2-21 | 2-Chlor-3-Fluorphenyl |
| 2-22 | 2-Chlor-4-Fluorphenyl |
| 2-23 | 2-Pyridyl [*] |
| 2-24 | 3-Pyridyl [*] |
| 2-25 | 4-Pyridyl [*] |
| 2-26 | 2-Thienyl [*] |
| 2-27 | 3-Thienyl [*] |
| 2-28 | Thiazol-4-yl [*] |
| 2-29 | Thiazol-5-yl [*] |
| 2-30 | Oxazol-4-yl [*] |
| 2-31 | Oxazol-5-yl [*] |
| 2-32 | 2-Furyl [*] |

**Tabelle 3**

| Zeile | Substituent B |
|---|---|
| 3-1 | 2-Chlor-thien-3-yl |
| 3-2 | 4-Chlor-thien-3-yl |
| 3-3 | 5-Chlor-thien-3-yl |
| 3-4 | 2,5-Dichlor-thien-3-yl |
| 3-5 | 2,4,5-Trichlor-thien-3-yl |
| 3-6 | 2-Brom-thien-3-yl |
| 3-7 | 4-Brom-thien-3-yl |
| 3-8 | 5-Brom-thien-3-yl |
| 3-9 | 2,5-Dibrom-thien-3-yl |
| 3-10 | 2,4,5-Tribrom-thien-3-yl |
| 3-11 | 3-Brom-fur-2-yl [*] |
| 3-12 | 4-Brom-fur-2-yl [*] |
| 3-13 | 5-Brom-fur-2-yl [*] |
| 3-14 | 4,5-Dibrom-fur-2-yl [*] |
| 3-15 | 4-Chlor-fur-2-yl [*] |
| 3-16 | 5-Chlor-fur-2-yl [*] |
| 3-17 | 3,4-Dichlor-fur-2-yl [*] |
| 3-18 | 2-Chlor-thiazol-5-yl [*] |
| 3-19 | 4-Chlor-thiazol-5-yl [*] |
| 3-20 | 2,4-Dichlor-thiazol-5-yl [*] |
| 3-21 | 2-Brom-thiazol-5-yl [*] |
| 3-22 | 2-Chlor-thiazol-4-yl [*] |
| 3-23 | 2-Brom-thiazol-4-yl [*] |
| 3-24 | 1-Methyl-pyrazol-4-yl [*] |
| 3-25 | 1,3-Dimethyl-pyrazol-4-yl [*] |
| 3-26 | 1,5-Dimethyl-pyrazol-4-yl [*] |
| 3-27 | 1,3,5-Trimethylpyrazol-4-yl [*] |
| 3-28 | 1-Methyl-pyrazol-3-yl [*] |
| 3-29 | 1-Methyl-imidazol-4-yl [*] |
| 3-30 | 1,5-Dimethyl-imidazol-4-yl [*] |
| 3-31 | 1-Methyl-pyrazol-5-yl [*] |
| 3-32 | 1,2-Dimethyl-imidazol-5-yl [*] |
| 3-33 | 1,4-Dimethyl-imidazol-5-yl [*] |

**Tabelle 4**

| Zeile | Substituent A |
|---|---|
| 4-1 | Phenyl [*] |
| 4-2 | 2-Methylphenyl |
| 4-3 | 3-Methylphenyl |
| 4-4 | 4-Methylphenyl |
| 4-5 | 2-Methoxyphenyl |
| 4-6 | 3-Methoxyphenyl |
| 4-7 | 4-Methoxyphenyl |
| 4-8 | 2-Chlorphenyl |
| 4-9 | 3-Chlorphenyl |
| 4-10 | 4-Chlorphenyl |
| 4-11 | 2-Fluorphenyl |
| 4-12 | 3-Fluorphenyl |
| 4-13 | 4-Fluorphenyl |
| 4-14 | 2-Chlor-3-Methoxyphenyl |
| 4-15 | 2-Chlor-4-Methoxyphenyl - |
| 4-16 | 2,3-Dichlorphenyl |
| 4-17 | 2,4-Dichlorphenyl |
| 4-18 | 3,4-Dichlorphenyl |
| 4-19 | 2,3-Difluorphenyl |
| 4-20 | 2,4-Difluorphenyl |
| 4-21 | 2-Chlor-3-Fluorphenyl |
| 4-22 | 2-Chlor-4-Fluorphenyl |
| 4-23 | 2-Pyridyl [*] |
| 4-24 | 3-Pyridyl [*] |
| 4-25 | 4-Pyridyl [*] |
| 4-26 | 2-Thienyl [*] |
| 4-27 | 3-Thienyl [*] |
| 4-28 | Thiazol-4-yl [*] |
| 4-29 | Thiazol-5-yl [*] |
| 4-30 | Oxazol-4-yl [*] |
| 4-31 | Oxazol-5-yl [*] |
| 4-32 | 2-Furyl [*] |

### Tabelle 5

Verbindungen der Formel I, in denen A für den Substituenten 1-1 der Tabelle 1 steht und B jeweils einem Substituenten einer Zeile der Tabelle 2 entspricht.

### Tabelle 6

Verbindungen der Formel I, in denen A für den Substituenten 1-2 der Tabelle 1 steht und B jeweils einem Substituenten einer Zeile der Tabelle 2 entspricht.

### Tabelle 7

Verbindungen der Formel I, in denen A für den Substituenten 1-3 der Tabelle 1 steht und B jeweils einem Substituenten einer Zeile der Tabelle 2 entspricht.

### Tabelle 8

Verbindungen der Formel I, in denen A für den Substituenten 1-4 der Tabelle 1 steht und B jeweils einem Substituenten einer Zeile der Tabelle 2 entspricht.

### Tabelle 9

Verbindungen der Formel I, in denen A für den Substituenten 1-5 der Tabelle 1 steht und B jeweils einem Substituenten einer Zeile der Tabelle 2 entspricht.

### Tabelle 10

Verbindungen der Formel I, in denen A für den Substituenten 1-6 der Tabelle 1 steht und B jeweils einem Substituenten einer Zeile der Tabelle 2 entspricht.

### Tabelle 11

Verbindungen der Formel I, in denen A für den Substituenten 1-7 der Tabelle 1 steht und B jeweils einem Substituenten einer Zeile der Tabelle 2 entspricht.

### Tabelle 12

Verbindungen der Formel I, in denen A für den Substituenten 1-8 der Tabelle 1 steht und B jeweils einem Substituenten einer Zeile der Tabelle 2 entspricht.

### Tabelle 13

Verbindungen der Formel I, in denen A für den Substituenten 1-9 der Tabelle 1 steht und B jeweils einem Substituenten einer Zeile der Tabelle 2 entspricht.

### Tabelle 14

Verbindungen der Formel I, in denen A für den Substituenten 1-10 der Tabelle 1 steht und B jeweils einem Substituenten einer Zeile der Tabelle 2 entspricht.

### Tabelle 15

Verbindungen der Formel I, in denen A für den Substituenten 1-11 der Tabelle 1 steht und B jeweils einem Substituenten einer Zeile der Tabelle 2 entspricht.

### Tabelle 16

Verbindungen der Formel I, in denen A für den Substituenten 1-12 der Tabelle 1 steht und B jeweils einem Substituenten einer Zeile der Tabelle 2 entspricht.

### Tabelle 17

Verbindungen der Formel I, in denen A für den Substituenten 1-13 der Tabelle 1 steht und B jeweils einem Substituenten einer Zeile der Tabelle 2 entspricht.

### Tabelle 18

Verbindungen der Formel I, in denen A für den Substituenten 1-14 der Tabelle 1 steht und B jeweils einem Substituenten einer Zeile der Tabelle 2 entspricht.

### Tabelle 19

Verbindungen der Formel I, in denen A für den Substituenten 1-15 der Tabelle 1 steht und B jeweils einem Substituenten einer Zeile der Tabelle 2 entspricht.

### Tabelle 20

Verbindungen der Formel I, in denen A für den Substituenten 1-16 der Tabelle 1 steht und B jeweils einem Substituenten einer Zeile der Tabelle 2 entspricht.

### Tabelle 21

Verbindungen der Formel I, in denen A für den Substituenten 1-17 der Tabelle 1 steht und B jeweils einem Substituenten einer Zeile der Tabelle 2 entspricht.

### Tabelle 22

Verbindungen der Formel I, in denen A für den Substituenten 1-18 der Tabelle 1 steht und B jeweils einem Substituenten einer Zeile der Tabelle 2 entspricht.

### Tabelle 23

Verbindungen der Formel I, in denen A für den Substituenten 1-19 der Tabelle 1 steht und B jeweils einem Substituenten einer Zeile der Tabelle 2 entspricht.

### Tabelle 24

Verbindungen der Formel I, in denen A für den Substituenten 1-20 der Tabelle 1 steht und B jeweils einem Substituenten einer Zeile der Tabelle 2 entspricht.

### Tabelle 25

Verbindungen der Formel I, in denen A für den Substituenten 1-21 der Tabelle 1 steht und B jeweils einem Substituenten einer Zeile der Tabelle 2 entspricht.

### Tabelle 26

Verbindungen der Formel I, in denen A für den Substituenten 1-22 der Tabelle 1 steht und B jeweils einem Substituenten einer Zeile der Tabelle 2 entspricht.

### Tabelle 27

Verbindungen der Formel I, in denen A für den Substituenten 1-23 der Tabelle 1 steht und B jeweils einem Substituenten einer Zeile der Tabelle 2 entspricht.

### Tabelle 28

Verbindungen der Formel I, in denen A für den Substituenten 1-24 der Tabelle 1 steht und B jeweils einem Substituenten einer Zeile der Tabelle 2 entspricht.

### Tabelle 29

Verbindungen der Formel I, in denen A für den Substituenten 1-25 der Tabelle 1 steht und B jeweils einem Substituenten einer Zeile der Tabelle 2 entspricht.

### Tabelle 30

Verbindungen der Formel I, in denen A für den Substituenten 1-26 der Tabelle 1 steht und B jeweils einem Substituenten einer Zeile der Tabelle 2 entspricht.

### Tabelle 31

Verbindungen der Formel I, in denen A für den Substituenten 1-27 der Tabelle 1 steht und B jeweils einem Substituenten einer Zeile der Tabelle 2 entspricht.

### Tabelle 32

Verbindungen der Formel I, in denen A für den Substituenten 1-28 der Tabelle 1 steht und B jeweils einem Substituenten einer Zeile der Tabelle 2 entspricht.

### Tabelle 33

Verbindungen der Formel I, in denen A für den Substituenten 1-29 der Tabelle 1 steht und B jeweils einem Substituenten einer Zeile der Tabelle 2 entspricht.

### Tabelle 34

Verbindungen der Formel I, in denen A für den Substituenten 1-30 der Tabelle 1 steht und B jeweils einem Substituenten einer Zeile der Tabelle 2 entspricht.

### Tabelle 35

Verbindungen der Formel I, in denen A für den Substituenten 1-31 der Tabelle 1 steht und B jeweils einem Substituenten einer Zeile der Tabelle 2 entspricht.

### Tabelle 36

Verbindungen der Formel I, in denen A für den Substituenten 1-32 der Tabelle 1 steht und B jeweils einem Substituenten einer Zeile der Tabelle 2 entspricht.

### Tabelle 37

Verbindungen der Formel I, in denen A für den Substituenten 1-33 der Tabelle 1 steht und B jeweils einem Substituenten einer Zeile der Tabelle 2 entspricht.

### Tabelle 38

Verbindungen der Formel I, in denen B für den Substituenten 3-1 der Tabelle 3 steht und A jeweils einem Substituenten einer Zeile der Tabelle 4 enstpricht.

### Tabelle 39

Verbindungen der Formel I, in denen B für den Substituenten 3-2 der Tabelle 3 steht und A jeweils einem Substituenten einer Zeile der Tabelle 4 enstpricht.

### Tabelle 40

Verbindungen der Formel I, in denen B für den Substituenten 3-3 der Tabelle 3 steht und A jeweils einem Substituenten einer Zeile der Tabelle 4 enstpricht.

### Tabelle 41

Verbindungen der Formel I, in denen B für den Substituenten 3-4 der Tabelle 3 steht und A jeweils einem Substituenten einer Zeile der Tabelle 4 enstpricht.

### Tabelle 42

Verbindungen der Formel I, in denen B für den Substituenten 3-5 der Tabelle 3 steht und A jeweils einem Substituenten einer Zeile der Tabelle 4 enstpricht.

### Tabelle 43

Verbindungen der Formel I, in denen B für den Substituenten 3-6 der Tabelle 3 steht und A jeweils einem Substituenten einer Zeile der Tabelle 4 enstpricht.

### Tabelle 44

Verbindungen der Formel I, in denen B für den Substituenten 3-7 der Tabelle 3 steht und A jeweils einem Substituenten einer Zeile der Tabelle 4 enstpricht.

### Tabelle 45

Verbindungen der Formel I, in denen B für den Substituenten 3-8 der Tabelle 3 steht und A jeweils einem Substituenten einer Zeile der Tabelle 4 enstpricht.

### Tabelle 46

Verbindungen der Formel I, in denen B für den Substituenten 3-9 der Tabelle 3 steht und A jeweils einem Substituenten einer Zeile der Tabelle 4 enstpricht.

### Tabelle 47

Verbindungen der Formel I, in denen B für den Substituenten 3-10 der Tabelle 3 steht und A jeweils einem Substituenten einer Zeile der Tabelle 4 enstpricht.

### Tabelle 48

Verbindungen der Formel I, in denen B für den Substituenten 3-11 der Tabelle 3 steht und A jeweils einem Substituenten einer Zeile der Tabelle 4 enstpricht.

### Tabelle 49

Verbindungen der Formel I, in denen B für den Substituenten 3-12 der Tabelle 3 steht und A jeweils einem Substituenten einer Zeile der Tabelle 4 enstpricht.

### Tabelle 50

Verbindungen der Formel I, in denen B für den Substituenten 3-13 der Tabelle 3 steht und A jeweils einem Substituenten einer Zeile der Tabelle 4 enstpricht.

### Tabelle 51

Verbindungen der Formel I, in denen B für den Substituenten 3-14 der Tabelle 3 steht und A jeweils einem Substituenten einer Zeile der Tabelle 4 enstpricht.

### Tabelle 52

Verbindungen der Formel I, in denen B für den Substituenten 3-15 der Tabelle 3 steht und A jeweils einem Substituenten einer Zeile der Tabelle 4 enstpricht.

### Tabelle 53

Verbindungen der Formel I, in denen B für den Substituenten 3-16 der Tabelle 3 steht und A jeweils einem Substituenten einer Zeile der Tabelle 4 enstpricht.

### Tabelle 54

Verbindungen der Formel I, in denen B für den Substituenten 3-17 der Tabelle 3 steht und A jeweils einem Substituenten einer Zeile der Tabelle 4 enstpricht.

### Tabelle 55

Verbindungen der Formel I, in denen B für den Substituenten 3-18 der Tabelle 3 steht und A jeweils einem Substituenten einer Zeile der Tabelle 4 enstpricht.

### Tabelle 56

Verbindungen der Formel I, in denen B für den Substituenten 3-19 der Tabelle 3 steht und A jeweils einem Substituenten einer Zeile der Tabelle 4 enstpricht.

### Tabelle 57

Verbindungen der Formel I, in denen B für den Substituenten 3-20 der Tabelle 3 steht und A jeweils einem Substituenten einer Zeile der Tabelle 4 enstpricht.

### Tabelle 58

Verbindungen der Formel I, in denen B für den Substituenten 3-21 der Tabelle 3 steht und A jeweils einem Substituenten einer Zeile der Tabelle 4 enstpricht.

### Tabelle 59

Verbindungen der Formel I, in denen B für den Substituenten 3-22 der Tabelle 3 steht und A jeweils einem Substituenten einer Zeile der Tabelle 4 enstpricht.

### Tabelle 60

Verbindungen der Formel I, in denen B für den Substituenten 3-23 der Tabelle 3 steht und A jeweils einem Substituenten einer Zeile der Tabelle 4 enstpricht.

### Tabelle 61

Verbindungen der Formel I, in denen B für den Substituenten 3-24 der Tabelle 3 steht und A jeweils einem Substituenten einer Zeile der Tabelle 4 eristpricht.

### Tabelle 62

Verbindungen der Formel I, in denen B für den Substituenten 3-25 der Tabelle 3 steht und A jeweils einem Substituenten einer Zeile der Tabelle 4 enstpricht.

### Tabelle 63

Verbindungen der Formel I, in denen B für den Substituenten 3-26 der Tabelle 3 steht und A jeweils einem Substituenten einer Zeile der Tabelle 4 enstpricht.

### Tabelle 64

Verbindungen der Formel I, in denen B für den Substituenten 3-27 der Tabelle 3 steht und A jeweils einem Substituenten einer Zeile der Tabelle 4 enstpricht.

### Tabelle 65

Verbindungen der Formel I, in denen B für den Substituenten 3-28 der Tabelle 3 steht und A jeweils einem Substituenten einer Zeile der Tabelle 4 enstpricht.

### Tabelle 66

Verbindungen der Formel I, in denen B für den Substituenten 3-29 der Tabelle 3 steht und A jeweils einem Substituenten einer Zeile der Tabelle 4 enstpricht.

### Tabelle 67

Verbindungen der Formel I, in denen B für den Substituenten 3-30 der Tabelle 3 steht und A jeweils einem Substituenten einer Zeile der Tabelle 4 enstpricht.

### Tabelle 68

Verbindungen der Formel I, in denen B für den Substituenten 3-31 der Tabelle 3 steht und A jeweils einem Substituenten einer Zeile der Tabelle 4 enstpricht.

### Tabelle 69

Verbindungen der Formel I, in denen B für den Substituenten 3-32 der Tabelle 3 steht und A jeweils einem Substituenten einer Zeile der Tabelle 4 enstpricht.

### Tabelle 70

Verbindungen der Formel I, in denen B für den Substituenten 3-33 der Tabelle 3 steht und A jeweils einem Substituenten einer Zeile der Tabelle 4 enstpricht.

Die Verbindungen eignen sich als Fungizide. Sie zeichnen sich aus durch eine hervorragende Wirksamkeit gegen ein breites Spektrum von pflanzenpathogenen Pilzen aus der Klasse der Ascomyceten, Deuteromyceten, Oomyceten und Basidiomyceten, insbesondere aus der Klasse der Oomyceten. Sie sind zum Teil systemisch wirksam und können im Pflanzenschutz als Blatt-, Beiz- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbissen, sowie an den Samen dieser Pflanzen.

Speziell eignen sie sich zur Bekämpfung folgender Pflanzenkrankheiten:
- Alternaria Arten an Gemüse, Raps, Zuckerrüben und Obst und Reis , wie z.B.
   A.solani oder A. alternata an Kartoffeln und Tomaten,
- Aphanomyces Arten an Zuckerrüben und Gemüse,
- Ascochyta-Arten an Getreide and Gemüse,
- Bipolaris- und Drechslera Arten an Mais, Getreide, Reis und Rasen, wie z.B. D.maydis an Mais,
- Blumeria graminis (Echter Mehltau) an Getreide,
- Botrytis cinerea (Grauschimmel) an Erdbeeren, Gemüse, Blumen und Weinreben,
- Bremia lactucae an Salat,
- Cercospora Arten an Mais, Sojabohnen, Reis und Zuckerrüben,
- Cochliobolus Arten an Mais , Getreide, Reis, wie z.B. Cochliobolus sativus an Getreide, Cochliobolus miyabeanus an Reis,
- Colletotricum Arten an Sojabohnen und Baumwolle,
- Drechslera Arten, Pyrenophora Arten an Mais, Getreide, Reis und Rasen, wie z.B. D.teres an Gerste oder D. tritici-repentis an Weizen,
- Esca an Weinrebe, verursacht durch Phaeoacremonium chlamydosporium, Ph. Aleophilum, und Formitipora punctata (syn. Phellinus punctatus),
- Exserohilum Arten an Mais,
- Erysiphe cichoracearum und Sphaerotheca fuliginea an Gurkengewächsen,
- Fusarium und Verticillium Arten an verschiedenen Pflanzen wie z.B. F. graminearum oder F. culmorum an Getreide oder F. oxysporum an einer Vielzahl von Pflanzen wie z.B. Tomaten,
- Gaeumanomyces graminis an Getreide,
- Gibberella arten an Getreide und Reis (z.B. Gibberella fujikuroi an Reis),
- Grainstaining complex an Reis,
- Helminthosporium Arten an Mais und Reis,
- Michrodochium nivale an Getreide,
- Mycosphaerella Arten an Getreide, Bananen und Erdnüssen, wie z.B. M. graminicola an Weizen oder M.fijiensis an Bananen,
- Peronospora-Arten an Kohl und Zwiebelgewächsen, wie z.B. P. brassicae an Kohl oder P. destructor an Zwiebel,
- Phakopsara pachyrhizi und Phakopsara meibomiae an Sojabohnen,
- Phomopsis Arten an Sojabohnen und Sonnenblumen,
- Phytophthora infestans an Kartoffeln und Tomaten,
- Phytophthora Arten an verschiedenen Pflanzen wie z.B. P.capsici an Paprika,
- Plasmopara viticola an Weinreben,
- Podosphaera leucotricha an Apfel,
- Pseudocercosporella herpotrichoides an Getreide,
- Pseudoperonospora an verschiedenen Pflanzen wie z.B. P. cubensis an Gurke oder P. humili an Hopfen,
- Puccinia Arten an verschiedenen Pflanzen wie z.B. P. triticina, P. striformins, P. hordei oder P.graminis an Getreide, oder P. asparagi an Spargel,
- Pyricularia oryzae , Corticium sasakii , Sarocladium oryzae, S.attenuatum, Entyloma oryzae, an Reis,
- Pyricularia grisea an Rasen und Getreide,
- Pythium spp. an Rasen, Reis, Mais, Baumwolle, Raps, Sonnenblumen, Zuckerrüben, Gemüse und anderen Pflanzen wie z.B. P.ultiumum an verschiedenen Pflanzen, P. aphanidermatum an Rasen,
- Rhizoctonia-Arten an Baumwolle, Reis, Kartoffeln, Rasen, Mais, Raps, Kartoffeln, Zuckerrüben, Gemüse und an verschiedenen Pflanzen wie z.B. R.solani an Rüben und verschiedenen Pflanzen,
- Rhynchosporium secalis an Gerste, Roggen und Triticale,
- Sclerotinia Arten an Raps und Sonnenblumen,
- Septoria tritici und Stagonospora nodorum an Weizen,
- Erysiphe (syn. Uncinula) necator an Weinrebe,
- Setospaeria Arten an Mais und Rasen,
- Sphacelotheca reilinia an Mais,
- Thievaliopsis Arten an Sojabohnen und Baumwolle,
- Tilletia Arten an Getreide,
- Ustilago-Arten an Getreide, Mais und Zuckerrohr, wie z.B. U. maydis an Mais,
- Venturia-Arten (Schorf) an Äpfeln und Birnen wie. z.B. V. inaequalis an Apfel.

Insbesondere eignen sie sich zur Bekämpfung von Schadpilzen aus der Klasse der Peronosporomycetes (syn.Oomyceten), wie Peronospora-Arten, Phytophthora-Arten, Plasmopara viticola , Pseudoperonospora-Arten und Pythium-Arten.

Die Verbindungen eignen sich außerdem zur Bekämpfung von Schadpilzen im Materialschutz (z.B. Holz, Papier, Dispersionen für den Anstrich, Fasern bzw. Gewebe) und im Vorratsschutz. Im Holzschutz finden insbesondere folgende Schadpilze Beachtung: Ascomyceten wie Ophiostoma spp., Ceratocystis spp., Aureobasidium pullulans, Sclerophoma spp., Chaetomium spp., Humicola spp., Petriella spp., Trichurus spp.; Basidiomyceten wie Coniophora spp., Coriolus spp., Gloeophyllum spp., Lentinus spp., Pleurotus spp., Poria spp., Serpula spp. und Tyromyces spp., Deuteromyceten wie Aspergillus spp., Cladosporium spp., Penicillium spp., Trichoderma spp., Alternaria spp., Paecilomyces spp. und Zygomyceten wie Mucor spp., darüber hinaus im Materialschutz folgende Hefepilze: Candida spp. und Saccharomyces cerevisae.

Die Verbindungen werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 1 bis 1000 g/100 kg, vorzugsweise 5 bis 100 g/100 kg Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Kubikmeter behandelten Materials.

Die Verbindungen der Formel I können in verschiedenen Kristallmodifikationen vorliegen, die sich in der biologischen Wirksamkeit unterscheiden können. Sie sind ebenfalls Gegenstand der vorliegenden Erfindung.

Die Verbindungen können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln. Als Lösungsmittel / Hilfsstoffe kommen dafür im wesentlichen in Betracht:
- Wasser, aromatische Lösungsmittel (z.B. Solvesso Produkte, Xylol), Paraffine (z.B. Erdölfraktionen), Alkohole (z.B. Methanol, Butanol, Pentanol, Benzylalkohol), Ketone (z.B. Cyclohexanon, gamma-Butryolacton), Pyrrolidone (NMP, NOP), Acetate (Glykoldiacetat), Glykole, Dimethylfettsäureamide, Fettsäuren und Fettsäureester. Grundsätzlich können auch Lösungsmittelgemische verwendet werden,
- Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie LigninSulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate, Fettsäuren und sulfatierte Fettalkoholglykolether zum Einsatz, ferner Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Tristerylphenylpolyglykolether, Alkylarylpolyetheralkohole, Alkohol- und Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Isophoron, stark polare Lösungsmittel, z.B. Dimethylsulfoxid, N-Methylpyrrolidon oder Wasser in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90 % bis 100 %, vorzugsweise 95 % bis 100 % (nach NMR-Spektrum) eingesetzt.

### Beispiele für Formulierungen sind: 1. Produkte zur Verdünnung in Wasser

### A Wasserlösliche Konzentrate (SL, LS)

10 Gew.-Teile der Wirkstoffe werden mit 90 Gew.-Teilen Wasser oder einem wasserlöslichen Lösungsmittel gelöst. Alternativ werden Netzmittel oder andere Hilfsmittel zugefügt. Bei der Verdünnung in Wasser löst sich der Wirkstoff. Man erhält auf diese Weise eine Formulierung mit 10 Gew.-% Wirkstoffgehalt.

### B Dispergierbare Konzentrate (DC)

20 Gew.-Teile der Wirkstoffe werden in 70 Gew.-Teilen Cyclohexanon unter Zusatz von 10 Gew.-Teilen eines Dispergiermittels z.B. Polyvinylpyrrolidon gelöst. Bei Verdünnung in Wasser ergibt sich eine Dispersion. Der Wirkstoffgehalt beträgt 20 Gew.-%

### C Emulgierbare Konzentrate (EC)

15 Gew.-Teile der Wirkstoffe werden in 75 Gew.-Teilen Xylol unter Zusatz von Ca-Dodecylbenzolsulfonat und Ricinusölethoxylat (jeweils 5 Gew.-Teile) gelöst. Bei der Verdünnung in Wasser ergibt sich eine Emulsion. Die Formulierung hat 15 Gew.-% Wirkstoffgehalt.

### D Emulsionen (EW, EO, ES)

25 Gew.-Teile der Wirkstoffe werden in 35 Gew.-Teile Xylol unter Zusatz von Ca-Dodecylbenzolsulfonat und Ricinusölethoxylat (jeweils 5 Gew.-Teile) gelöst. Diese Mischung wird mittels einer Emulgiermaschine (z.B. Ultraturax) in 30 Gew.Teile Wasser gegeben und zu einer homogenen Emulsion gebracht. Bei der Verdünnung in Wasser ergibt sich eine Emulsion. Die Formulierung hat einen Wirkstoffgehalt von 25 Gew.-%.

### E Suspensionen (SC, OD, FS)

20 Gew.-Teile der Wirkstoffe werden unter Zusatz von 10 Gew.-Teilen Dispergier- und Netzmitteln und 70 Gew.-Teilen Wasser oder einem organischen Lösungsmittel in einer Rührwerkskugelmühle zu einer feinen Wirkstoffsuspension zerkleinert. Bei der Verdünnung in Wasser ergibt sich eine stabile Suspension des Wirkstoffs. Der Wirkstoffgehalt in der Formulierung beträgt 20 Gew.-% .

### F Wasserdispergierbare und wasserlösliche Granulate (WG, SG) 50 Gew.-Teile der Wirkstoffe werden unter Zusatz von 50 Gew-Teilen Dispergier- und Netzmitteln fein gemahlen und mittels technischer Geräte (z.B. Extrusion, Sprühturm, Wirbelschicht) als wasserdispergierbare oder wasserlösliche Granulate hergestellt. Bei der Verdünnung in Wasser ergibt sich eine stabile Dispersion oder Lösung des Wirkstoffs. Die Formulierung hat einen Wirkstoffgehalt von 50 Gew.-%.

G Wasserdispergierbare und wasserlösliche Pulver (WP, SP, SS, WS) 75 Gew.-Teile der Wirkstoffe werden unter Zusatz von 25 Gew.-Teilen Dispergier- und Netzmitteln sowie Kieselsäuregel in einer Rotor-Strator Mühle vermahlen. Bei der Verdünnung in Wasser ergibt sich eine stabile Dispersion oder Lösung des Wirkstoffs. Der Wirkstoffgehalt der Formulierung beträgt 75 Gew.-%.

### H Gelformulierungen (GF)

In einer Kugelmühle werden 20 Gew.-Teile der Wirkstoffe, 10 Gew.-Teile Dispergiermittel, 1 Gew.-Teil Quellmittel ("gelling agent") und 70 Gew.-Teile Wasser oder eines organischen Lösungsmittels zu einer feinen Suspension vermahlen. Bei der Verdünnung mit Wasser ergibt sich eine stabile Suspension mit 20 Gew.-% Wirkstoffgehalt.

### 2. Produkte für die Direktapplikation

### I Stäube (DP, DS)

5 Gew.-Teile der Wirkstoffe werden fein gemahlen und mit 95 Gew.-Teilen feinteiligem Kaolin innig vermischt. Man erhält dadurch ein Stäubemittel mit 5 Gew.-% Wirkstoffgehalt.

### J Granulate (GR, FG, GG, MG)

0,5 Gew-Teile der Wirkstoffe werden fein gemahlen und mit 99,5 Gewichtsteilen Trägerstoffe verbunden. Gängige Verfahren sind dabei die Extrusion, die Sprühtrocknung oder die Wirbelschicht. Man erhält dadurch ein Granulat für die Direktapplikation mit 0,5 Gew.-% Wirkstoffgehalt.

### K ULV- Lösungen (UL)

10 Gew.-Teile der Wirkstoffe werden in 90 Gew.-Teilen eines organischen Lösungsmittel z.B. Xylol gelöst. Dadurch erhält man ein Produkt für die Direktapplikation mit 10 Gew.-% Wirkstoffgehalt.

Für die Saatgutbehandlung werden üblicherweise wasserlösliche Konzentrate (LS), Suspensionen (FS), Stäube (DS), wasserdispergierbare und wasserlösliche Pulver (WS, SS), Emulsionen (ES), emulgierbare Konzentrate (EC) und Gelformulierungen (GF) verwendet. Diese Formulierungen können auf das Saatgut unverdünnt oder, bevorzugt, verdünnt angewendet werden. Die Anwendung kann vor der Aussaat erfolgen.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wässrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Netzmittel, Adjuvants, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:100 bis 100:1, bevorzugt 1:10 bis 10:1 zugemischt werden.

Als Adjuvants in diesem Sinne kommen insbesondere in Frage: organisch modifizierte Polysiloxane, z.B. Break Thru S 240^{®}; Alkoholalkoxylate, z. B. Atplus 245^{®}, Atplus MBA 1303^{®}, Plurafac LF 300^{®} und Lutensol ON 30^{®}; EO-PO-Blockpolymerisate, z. B. Pluronic RPE 2035^{®} und Genapol B^{®}; Alkoholethoxylate, z. B. Lutensol XP 80^{®}; und Natriumdioctylsulfosuccinat, z. B. Leophen RA^{®}.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel mit einem oder mehreren weiteren Wirkstoffen, insbesondere Fungiziden, kann beispielsweise in vielen Fällen das Wirkungsspektrum verbreitert werden oder Resistenzentwicklungen vorgebeugt werden. In vielen Fällen erhält man dabei synergistische Effekte.

Ein weiterer Gegenstand der vorliegenden Erfindung ist eine Kombination aus mindestens einem Azolylmethyloxiran der Formel I, insbesondere einem in der vorliegenden Beschreibung als bevorzugt offenbartes Azolylmethyloxiran und/oder einem landwirtschaftlich verträglichen Salz davon und mindestens einem weiteren fungiziden, insektiziden, herbiziden und/oder wachstumsregulierenden Wirkstoff, wobei eine synergistische Wirkung auftreten kann.

Noch ein Gegenstand der vorliegenden Erfindung ist ein pestizides Mittel, umfassend mindestens eine Verbindung der Formel I, insbesondere eine in der vorliegenden Beschreibung als bevorzugt beschriebene Verbindung der Formel I und/oder ein landwirtschaftlich verträgliches Säureadditions- oder Metallsalz davon und mindestens einen festen oder flüssigen Trägerstoff. Ein solches pestizides Mittel kann mindestens einen weiteren fungiziden, insektiziden und/oder herbiziden Wirkstoff enthalten, wobei auch eine synergistische Wirkung auftreten kann.

Die folgende Liste L von Fungiziden, mit denen die erfindungsgemäßen Verbindungen gemeinsam angewendet werden können, soll die Kombinationsmöglichkeiten erläutern, nicht aber einschränken:

### Liste L:

### Strobilurine

Azoxystrobin, Dimoxystrobin, Enestroburin, Fluoxastrobin, Kresoxim-methyl, Methominostrobin, Orysastrobin, Picoxystrobin, Pyraclostrobin, Pyribencarb, Trifloxystrobin, 2-(2-(6-(3-Chlor-2-methyl-phenoxy)-5-fluor-pyrimidin-4-yloxy)-phenyl)-2-methoxyimino-N-methyl-acetamid, 2-(ortho-((2,5-Dimethylphenyl-oxymethylen)phenyl)-3-methoxy-acrylsäuremethylester, 3-Methoxy-2-(2-(N-(4-methoxy-phenyl)-cyclopropancarboximidoylsulfanylmethyl)-phenyl)-acrylsäuremethylester;

### Carbonsäureamide

- Carbonsäureanilide: Benalaxyl, Benalaxyl-M, Benodanil, Bixafen, Boscalid, Carboxin, Fenfuram, Fenhexamid, Flutolanil, Furametpyr, Isotianil, Kiralaxyl, Mepronil, Metalaxyl, Ofurace , Oxadixyl, Oxycarboxin , Penthiopyrad, Tecloftalam, Thifluzamide, Tiadinil, 2-Amino-4-methyl-thiazol-5-carbonsäureanilid, 2-Chlor-N-(1,1,3-trimethyl-indan-4-yl)-nicotinamid, N-(3',4'-Dichlor-5-fluor-biphenyl-2-yl)-3-difluormethyl-1-methyl-1 H-pyrazol-4-carbonsäureamid, 5-Fluor-1,3-dimethyl-1 H-pyrazol-4-carbonsäure [2-(1,3-dimethyl-butyl)-phenyl]-amid, N-(4'-Chlor-3',5-difluor-biphenyl-2-yl)-3-difluormethyl-1-methyl-1 H-pyrazol-4-carbonsäureamid, N-(4'-Chlor-3',5-difluorbiphenyl-2-yl)-3-trifluormethyl-1-methyl-1 H-pyrazol-4-carbonsäureamid, N-(3',4'-Dichlor-5-fluor-biphenyl-2-yl)-3-trifluormethyl-1-methyl-1 H-pyrazol-4-carbonsäureamid, N-(3',5-Difluor-4'-methyl-biphenyl-2-yl)-3-difluormethyl-1-methyl-1 H-pyrazol-4-carbonsäureamid, N-(3',5-Difluor-4'-methyl-biphenyl-2-yl)-3-trifluormethyl-1-methyl-1 H-pyrazol-4-carbonsäureamid, N-(2-Bicyclopropyl-2-yl-phenyl)-3-difluormethyl-1-methyl-1 H-pyrazol-4-carbonsäureamid, N-(cis-2-Bicyclopropyl-2-yl-phenyl)-3-difluormethyl-1-methyl-1 H-pyrazol-4-carbonsäureamid, N-(trans-2-Bicyclopropyl-2-yl-phenyl)-3-difluormethyl-1-methyl-1 H-pyrazol-4-carbonsäureamid,
- Carbonsäuremorpholide: Dimethomorph, Flumorph;
- Benzoesäureamide: Flumetover, Fluopicolide, Fluopyram, Zoxamide, N-(3-Ethyl-3,5-5trimethyl-cyclohexyl)-3-formylamino-2-hydroxy-benzamid;
- Sonstige Carbonsäureamide: Carpropamid, Diclocymet, Mandipropamid, Oxytetracyclin, Silthiofam, N-(6-methoxy-pyridin-3-yl)cyclopropancarbonsäureamid;

### Azole

- Triazole: Azaconazole, Bitertanol, Bromuconazole, Cyproconazole, Difenoconazole, Diniconazole, Diniconazole-M, Epoxiconazole, Fenbuconazole, Fluquinconazole, Flusilazole, Flutriafol, Hexaconazol, Imibenconazole, lpconazole, Metconazol, Myclobutanil, Oxpoconazol, Paclobutrazol, Penconazole, Propiconazole, Prothioconazole, Simeconazole, Tebuconazole, Tetraconazole, Triadimefon, Triadimenol, Triticonazole, Uniconazol, 1-(4-Chlor-phenyl)-2-([1,2,4]triazol-1-yl)-cycloheptanol;
- Imidazole: Cyazofamid, Imazalil, Imazalil-sulfat, Pefurazoate, Prochloraz, Triflumizole;
- Benzimidazole: Benomyl, Carbendazim , Fuberidazole , Thiabendazole;
- Sonstige: Ethaboxam, Etridiazole, Hymexazole, 1-(4-Chlor-phenyl)-1-(propin-2-yloxy)-3-(4-(3,4-dimethoxy-phenyl)-isoxazol-5-yl)-propan-2-on;

### Stickstoffhaltige Heterocyclylverbindungen

- Pyridine: Fluazinam, Pyrifenox, 3-[5-(4-Chlor-phenyl)-2,3-dimethyl-isoxazolidin-3-yl]-pyridin, 2,3,5,6-Tetrachlor-4-methansulfonyl-pyridin, 3,4,5-Trichlor-pyridin-2,6-dicarbonitril, N-(1-(5-Brom-3-chlor-pyridin-2-yl)-ethyl)-2,4-dichlor-nicotinamid, N-((5-Brom-3-chlor-pyridin-2-yl)-methyl)-2,4-dichlor-nicotinamid;
- Pyrimidine: Bupirimate, Cyprodinil, Diflumetorim, Fenarimol, Ferimzone, Mepanipyrim, Nitrapyrin, Nuarimol, Pyrimethanil;
- Pyrrole: Fludioxonil, Fenpiclonil;
- Morpholine: Aldimorph, Dodemorph, Dodemorph-Acetat, Fenpropimorph, Tridemorph;
- Dicarboximide: Fluoroimid, Iprodione, Procymidone, Vinclozolin;
- sonstige: Acibenzolar-S-methyl, Amisulbrom, Anilazin, Blasticidin-S, Captafol, Captan, Chinomethionat, Dazomet, Debacarb, Diclomezine, Difenzoquat, Difenzoquat-methylsulphat, Famoxadone, Fenamidone, Fenoxanil, Fenpropidin, Folpet, Octhilinone, Oxolinsäure, Piperalin, Probenazole, Proquinazid, Pyroquilon, Quinoxyfen, Triazoxid, Tricyclazole, Triforine, 5-Chlor-7-(4-methyl-piperidin-1-yl)-6-(2,4,6-trifluor-phenyl)-[1,2,4]triazolo[1,5-a]pyrimidin, 2-Butoxy-6-iodo-3-propylchromen-4-on;

### Carbamate und Dithiocarbamate

- Thio- und Dithiocarbamate: Ferbam, Mancozeb, Maneb, Metam, Methasulphocarb, Metiram, Propineb, Thiram, Zineb, Ziram;
- Carbamate: Diethofencarb, Benthiavalicarb, Iprovalicarb, Propamocarb, Propamocarb hydrochlorid, Valiphenal, N-(1-(1-(4-Cyanophenyl)ethansulfonyl)-but-2-yl)carbaminsäure-(4-fluorophenyl)ester;

### Sonstige Fungizide

- Guanidine: Dodine, Dodine freie Base, Guazatine, Guazatine-Acetat, Iminoctadine, Iminoctadine-Triacetat, Iminoctadine-tris(albesilat);
- Antibiotika: Kasugamycin, Kasugamycin-hydrochlorid-Hydrat, Polyoxine, Streptomycin, Validamycin A;
- Nitrophenylderivate:
   Binapacryl, Dicloran, Dinobuton, Dinocap, Nitrothal-isopropyl, Tecnazen;
- Organometallverbindungen: Fentin Salze wie beispielsweise Fentin-Acetat, Fentin-Chlorid, Fentin-Hydroxid;
- Schwefelhaltige Heterocyclylverbindungen: Isoprothiolane, Dithianon;
- Organophosphorverbindungen: Edifenphos, Fosetyl, Fosetyl-aluminium, Iprobenfos, Pyrazophos, Tolclofos-methyl;
- Organochlorverbindungen: Chlorothalonil, Dichlofluanid, Dichlorophen, Flusulfamide, Hexachlorbenzene, Pencycuron, Pentachlorophenol und dessen Salze, Phthalid, Quintozene, Thiophanate-Methyl, Tolylfluanid, N-(4-Chlor-2-nitro-phenyl)-N-ethyl-4-methyl-benzolsulfonamid;
- Anorganische Wirkstoffe: Phosphorige Säure und ihre Salze, Schwefel, Bordeaux Brühe, Kupfersalze wie beispielsweise Kupferacetat, Kupferhydroxid, Kupferoxychlorid, basisches Kupfersulfat;
- Sonstige: Biphenyl, Bronopol, Cyflufenamid, Cymoxanil, Diphenylamin, Metrafenone, Mildiomycin, Oxin-Kupfer, Prohexadione-Calcium, Spiroxamine, Tolylfluanid, N-(Cyclopropylmethoxyimino-(6-difluormethoxy-2,3-difluor-phenyl)-methyl)-2-phenyl acetamid, N'-(4-(4-Chlor-3-trifluormethyl-phenoxy)-2,5-dimethyl-phenyl)-N-ethyl-N-methylformamidin, N'-(4-(4-Fluor-3-trifluormethyl-phenoxy)-2,5-dimethyl-phenyl)-N-ethyl-N-methylformamidin, N'-(2-Methyl-5-trifluormethyl-4-(3-trimethylsilanyl-propoxy)-phenyl)-N-ethyl-N-methylformamidin, N'-(5-Difluormethyl-2-methyl-4-(3-trimethylsilanyl-propoxy)-phenyl)-N-ethyl-N-methylformamidin.

Demgemäß betrifft die vorliegenden Erfindung ferner die in der Tabelle B aufgeführten Zusammensetzungen, wobei jeweils eine Zeile der Tabelle B einer fungiziden Zusammensetzung entspricht, umfassend eine Verbindung der Formel I (Komponente 1), welche vorzugsweise eine der hierin als bevorzugt beschriebenen Verbindungen ist, und den jeweils in der betreffenden Zeile angegebenen weiteren Wirkstoff (Komponente 2). Gemäß einer Ausgestaltung der Erfindung ist Komponente 1 in jeder Zeile der Tabelle B jeweils eine der in den Tabellen 1 bis xxxx spezifisch individualisierten Verbindungen der Formel I.

**Tabelle B**

| Zeile | Komponente 1 | Komponente 2 |
|---|---|---|
| B-1 | eine Verbindung der Formel I | Azoxystrobin |
| B-2 | eine Verbindung der Formel I | Dimoxystrobin |
| B-3 | eine Verbindung der Formel I | Enestroburin |
| B-4 | eine Verbindung der Formel I | Fluoxastrobin |
| B-5 | eine Verbindung der Formel I | Kresoxim-methyl |
| B-6 | eine Verbindung der Formel I | Metominostrobin |
| B-7 | eine Verbindung der Formel I | Orysastrobin |
| B-8 | eine Verbindung der Formel I | Picoxystrobin |
| B-9 | eine Verbindung der Formel I | Pyractostrobin |
| B-10 | eine Verbindung der Formel I | Pyribencarb |
| B-11 | eine Verbindung der Formel I | Trifloxystrobin |
| B-12 | eine Verbindung der Formel I | 2-(2-(6-(3-Chlor-2-methyl-phenoxy)-5-fluor-pyrimidin-4-yloxy)-phenyl)-2-methoxyimino-N-methyl-acetamid |
| B-13 | eine Verbindung der Formel I | 2-(ortho-((2,5-Dimethylphenyl-oxymethylen)phenyl)-3-methoxy-acrylsäuremethylester |
| B-14 | eine Verbindung der Formel I | 3-Methoxy-2-(2-(N-(4-methoxy-phenyl)-cyclopropancarboximidoylsulfanylmethyl)-phenyl)-acrylsäuremethylester |
| B-15 | eine Verbindung der Formel I | Benalaxyl |
| B-16 | eine Verbindung der Formel I | Benalaxyl-M |
| B-17 | eine Verbindung der Formel I | Benodanil |
| B-18 | eine Verbindung der Formel I | Bixafen |
| B-19 | eine Verbindung der Formel I | Boscalid |
| B-20 | eine Verbindung der Formel I | Carboxin |
| B-21 | eine Verbindung der Formel I | Fenfuram |
| B-22 | eine Verbindung der Formel I | Fenhexamid |
| B-23 | eine Verbindung der Formel I | Flutolanil |
| B-24 | eine Verbindung der Formel I | Furametpyr |
| B-25 | eine Verbindung der Formel I | Isotianil |
| B-26 | eine Verbindung der Formel I | Kiralaxyl |
| B-27 | eine Verbindung der Formel I | Mepronil |
| B-28 | eine Verbindung der Formel I | Metalaxyl |
| B-29 | eine Verbindung der Formel I | Ofurace |
| B-30 | eine Verbindung der Formel I | Oxadixyl |
| B-31 | eine Verbindung der Formel I | Oxycarboxin |
| B-32 | eine Verbindung der Formel I | Penthiopyrad |
| B-33 | eine Verbindung der Formel I | Thifluzamide |
| B-34 | eine Verbindung der Formel I | Tecloftalam |
| B-35 | eine Verbindung der Formel I | Tiadinil |
| B-36 | eine Verbindung der Formel I | 2-Amino-4-methyl-thiazol-5-carbonsäure-anilid |
| B-37 | eine Verbindung der Formel I | 2-Chlor-N-(1,1,3-trimethyl-indan-4-yl)-ncotinamid |
| B-38 | eine Verbindung der Formel I | N-(3',4'-Dichlor-5-fluor-biphenyl-2-yl)-3-difluormethyl-1-methyl-1 H-pyrazol-4-carbonsäureamid |
| B-39 | eine Verbindung der Formel I | 5-Fluor-1,3-dimethyl-1 H-pyrazol-4-carbonsäure [2-(1 ,3-dimethyl-butyl)-phenyl]-amid |
| B-40 | eine Verbindung der Formel I | N-(4'-Chlor-3',5-difluor-biphenyl-2-yl)-3-difluormethyl-1-methyl-1H-pyrazol-4-carbonsäureamid |
| B-41 | eine Verbindung der Formel I | N-(4'-Chlor-3',5-difluor-biphenyl-2-yl)-3-trifluormethyl-1-methyl-1H-pyrazol-4-carbonsäureamid |
| B-42 | eine Verbindung der Formel I | N-(3',4'-Dichlor-5-fluor-biphenyl-2-yl)-3-trifluormethyl-1-methyl-1H-pyrazol-4-carbonsäureamid |
| B-43 | eine Verbindung der Formel I | N-(3',5-Difluor-4'-methyl-biphenyl-2-yl)-3-difluormethyl-1-methyl-1H-pyrazol-4-carbonsäureamid |
| B-44 | eine Verbindung der Formel I | N-(3',5-Difluor-4'-methyl-biphenyl-2-yl)-3-trifluormethyl-1-methyl-1H-pyrazol-4-carbonsäureamid |
| B-45 | eine Verbindung der Formel I | N-(2-Bicyclopropyl-2-yl-phenyl)-3-difluormethyl-1-methyl-1H-pyrazol-4-carbonsäureamid |
| B-46 | eine Verbindung der Formel I | N-(cis-2-Bicyclopropyl-2-yl-phenyl)-3-difluormethyl-1-methyl-1H-pyrazol-4-carbonsäureamid |
| B-47 | eine Verbindung der Formel I | N-(trans-2-Bicyclopropyl-2-yl-phenyl)-3-difluormethyl-1-methyl-1H-pyrazol-4-carbonsäureamid |
| B-48 | eine Verbindung der Formel I | Dimethomorph |
| B-49 | eine Verbindung der Formel I | Flumorph |
| B-50 | eine Verbindung der Formel I | Flumetover |
| B-51 | eine Verbindung der Formel I | Fluopicolide (Picobenzamid) |
| B-52 | eine Verbindung der Formel I | Fluopyram |
| B-53 | eine Verbindung der Formel I | Zoxamide |
| B-54 | eine Verbindung der Formel I | N-(3-Ethyl-3,5-5trimethyl-cyclohexyl)-3-formylamino-2-hydroxy-benzamid |
| B-55 | eine Verbindung der Formel I | Carpropamid |
| B-56 | eine Verbindung der Formel I | Diclocymet |
| B-57 | eine Verbindung der Formel I | Mandipropamid |
| B-58 | eine Verbindung der Formel I | Oxytetracyclin |
| B-59 | eine Verbindung der Formel I | Silthiofam |
| B-60 | eine Verbindung der Formel I | N-(6-methoxy-pyridin-3-yl)cyclopropancarbonsäureamid |
| B-61 | eine Verbindung der Formel I | Azaconazole |
| B-62 | eine Verbindung der Formel I | Bitertanol |
| B-63 | eine Verbindung der Formel I | Bromuconazole |
| B-64 | eine Verbindung der Formel I | Cyproconazole |
| B-65 | eine Verbindung der Formel I | Difenoconazole |
| B-66 | eine Verbindung der Formel I | Diniconazole |
| B-67 | eine Verbindung der Formel I | Diniconazole-M |
| B-68 | eine Verbindung der Formel I | Enilconazole |
| B-69 | eine Verbindung der Formel I | Epoxiconazole |
| B-70 | eine Verbindung der Formel I | Fenbuconazole |
| B-71 | eine Verbindung der Formel I | Flusilazole |
| B-72 | eine Verbindung der Formel I | Fluquinconazole |
| B-73 | eine Verbindung der Formel I | Flutriafol |
| B-74 | eine Verbindung der Formel I | Hexaconazol |
| B-75 | eine Verbindung der Formel I | Imibenconazole |
| B-76 | eine Verbindung der Formel I | Ipconazole |
| B-77 | eine Verbindung der Formel I | Metconazol |
| B-78 | eine Verbindung der Formel I | Myclobutanil |
| B-79 | eine Verbindung der Formel I | Oxpoconazol |
| B-80 | eine Verbindung der Formel I | Paclobutrazol |
| B-81 | eine Verbindung der Formel I | Penconazole |
| B-82 | eine Verbindung der Formel I | Propiconazole |
| B-83 | eine Verbindung der Formel I | Prothioconazole |
| B-84 | eine Verbindung der Formel I | Simeconazole |
| B-85 | eine Verbindung der Formel I | Tebuconazole |
| B-86 | eine Verbindung der Formel I | Tetraconazole |
| B-87 | eine Verbindung der Formel I | Triadimenol |
| B-88 | eine Verbindung der Formel I | Triadimefon |
| B-89 | eine Verbindung der Formel I | Triticonazole |
| B-90 | eine Verbindung der Formel I | Uniconazol |
| B-91 | eine Verbindung der Formel I | 1-(4-Chlor-phenyl)-2-([1,2,4]triazol-1-yl)-cycloheptanol |
| B-92 | eine Verbindung der Formel I | Cyazofamid |
| B-93 | eine Verbindung der Formel I | Imazalil |
| B-94 | eine Verbindung der Formel I | Imazalil-sulfat |
| B-95 | eine Verbindung der Formel I | Pefurazoate |
| B-96 | eine Verbindung der Formel I | Prochloraz |
| B-97 | eine Verbindung der Formel I | Triflumizole |
| B-98 | eine Verbindung der Formel I | Benomyl |
| B-99 | eine Verbindung der Formel I | Carbendazim |
| B-100 | eine Verbindung der Formel I | Fuberidazole |
| B-101 | eine Verbindung der Formel I | Thiabendazole |
| B-102 | eine Verbindung der Formel I | Ethaboxam |
| B-103 | eine Verbindung der Formel I | Etridiazole |
| B-104 | eine Verbindung der Formel I | Hymexazole |
| B-105 | eine Verbindung der Formel I | Fluazinam |
| B-106 | eine Verbindung der Formel I | Pyrifenox |
| B-107 | eine Verbindung der Formel I | 1-(4-Chlor-phenyl)-1-(propin-2-yloxy)-3-(4-(3,4-dimethoxy-phenyl)-isoxazol-5-yl)-propan-2-on |
| B-108 | eine Verbindung der Formel I | 3-[5-(4-Chlor-phenyl)-2,3-dimethyl-isoxa-3-[5-(4-Chlor-phenyl)-2,3-dimethyl-isoxa-zolidin-3-yl]-pyridin |
| B-109 | eine Verbindung der Formel I | 2,3,5,6-Tetrachlor-4-methansulfonyl-pyridin |
| B-110 | eine Verbindung der Formel I | 3,4,5-Trichlor-pyridin-2,6-dicarbonitril |
| B-111 | eine Verbindung der Formel I | N-(1-(5-Brom-3-chlor-pyridin-2-yl)-ethyl)-2,4-dichlor-nicotinamid |
| B-112 | eine Verbindung der Formel I | N-((5-Brom-3-chlor-pyridin-2-yl)-methyl)-2,4-dichlor-nicotinamid |
| B-113 | eine Verbindung der Formel I | Bupirimate |
| B-114 | eine Verbindung der Formel I | Cyprodinil |
| B-115 | eine Verbindung der Formel I | Diflumetorim |
| B-116 | eine Verbindung der Formel I | Ferimzone |
| B-117 | eine Verbindung der Formel I | Fenarimol |
| B-118 | eine Verbindung der Formel I | Mepanipyrim |
| B-119 | eine Verbindung der Formel I | Nitrapyrin |
| B-120 | eine Verbindung der Formel I | Nuarimol |
| B-121 | eine Verbindung der Formel I | Pyrimethanil |
| B-122 | eine Verbindung der Formel I | Fludioxonil |
| B-123 | eine Verbindung der Formel I | Fenpiclonil |
| B-124 | eine Verbindung der Formel I | Aldimorph |
| B-125 | eine Verbindung der Formel I | Dodemorph |
| B-126 | eine Verbindung der Formel I | Dodemorph-Acetat |
| B-127 | eine Verbindung der Formel I | Fenpropimorph |
| B-128 | eine Verbindung der Formel I | Tridemorph |
| B-129 | eine Verbindung der Formel I | Fluoroimid |
| B-130 | eine Verbindung der Formel I | Iprodione |
| B-131 | eine Verbindung der Formel I | Procymidone |
| B-132 | eine Verbindung der Formel I | Vinclozolin |
| B-133 | eine Verbindung der Formel I | Acibenzolar-S-methyl |
| B-134 | eine Verbindung der Formel I | Amisulbrom |
| B-135 | eine Verbindung der Formel I | Anilazin |
| B-136 | eine Verbindung der Formel I | Blasticidin-S |
| B-137 | eine Verbindung der Formel I | Captan |
| B-138 | eine Verbindung der Formel I | Captafol |
| B-139 | eine Verbindung der Formel I | Chinomethionat |
| B-140 | eine Verbindung der Formel I | Dazomet |
| B-141 | eine Verbindung der Formel I | Debacarb |
| B-142 | eine Verbindung der Formel I | Diclomezine |
| B-143 | eine Verbindung der Formel I | Difenzoquat |
| B-144 | eine Verbindung der Formel I | Difenzoquat-methylsulphat |
| B-145 | eine Verbindung der Formel I | Famoxadone |
| B-146 | eine Verbindung der Formel I | Fenamidone |
| B-147 | eine Verbindung der Formel I | Fenoxanil |
| B-148 | eine Verbindung der Formel I | Fenpropidin |
| B-149 | eine Verbindung der Formel I | Folpet |
| B-150 | eine Verbindung der Formel I | Octhilinone |
| B-151 | eine Verbindung der Formel I | Oxolinsäure |
| B-152 | eine Verbindung der Formel I | Piperalin |
| B-153 | eine Verbindung der Formel I | Probenazole |
| B-154 | eine Verbindung der Formel I | Proquinazid |
| B-155 | eine Verbindung der Formel I | Pyroquilon |
| B-156 | eine Verbindung der Formel I | Quinoxyfen |
| B-157 | eine Verbindung der Formel I | Triazoxid |
| B-158 | eine Verbindung der Formel I | Tricyclazole |
| B-159 | eine Verbindung der Formel I | Triforine |
| B-160 | eine Verbindung der Formel I | 5-Chlor-7-(4-methyl-piperidin-1 -yl)-6- (2,4,6-trifluor-phenyl)-[1,2,4]triazolo[1,5-a]pyrimidin |
| B-161 | eine Verbindung der Formel I | 2-Butoxy-6-iodo-3-propyl-chromen-4-on |
| B-162 | eine Verbindung der Formel I | Ferbam |
| B-163 | eine Verbindung der Formel I | Mancozeb |
| B-164 | eine Verbindung der Formel I | Maneb |
| B-165 | eine Verbindung der Formel I | Metiram |
| B-166 | eine Verbindung der Formel I | Metam |
| B-167 | eine Verbindung der Formel I | Methasulphocarb |
| B-168 | eine Verbindung der Formel I | Propineb |
| B-169 | eine Verbindung der Formel I | Thiram |
| B-170 | eine Verbindung der Formel I | Zineb |
| B-171 | eine Verbindung der Formel I | Ziram |
| B-172 | eine Verbindung der Formel I | Diethofencarb |
| B-173 | eine Verbindung der Formel I | Flubenthiavalicarb |
| B-174 | eine Verbindung der Formel I | Iprovalicarb |
| B-175 | eine Verbindung der Formel I | Propamocarb |
| B-176 | eine Verbindung der Formel I | Propamocarb-Hydrochlorid 3-(4-Chlor-phenyl)-3-(2-isopropoxycarbo- |
| B-177 | eine Verbindung der Formel I | nylamino-3-methyl-butyrylamino)-propionsäuremethylester |
| B-178 | eine Verbindung der Formel I | Valiphenal N-(1-(1-(4-Cyanophenyl)ethansulfonyl)- |
| B-179 | eine Verbindung der Formel I | but-2-yl)carbaminsäure-(4-fluorphenyl)ester |
| B-180 | eine Verbindung der Formel I | Dodine |
| B-181 | eine Verbindung der Formel I | Dodine freie Base |
| B-182 | eine Verbindung der Formel I | Iminoctadine |
| B-183 | eine Verbindung der Formel I | Iminoctadine Triacetat |
| B-184 | eine Verbindung der Formel I | Iminoctadine-tris(albesilat) |
| B-185 | eine Verbindung der Formel I | Guazatine |
| B-186 | eine Verbindung der Formel I | Guazatine Acetat |
| B-187 | eine Verbindung der Formel I | Kasugamycin |
| B-188 | eine Verbindung der Formel I | Kasugamycin-hydrochlorid-Hydrat |
| B-189 | eine Verbindung der Formel I | Polyoxine |
| B-190 | eine Verbindung der Formel I | Streptomycin |
| B-191 | eine Verbindung der Formel I | Validamycin A |
| B-192 | eine Verbindung der Formel I | Binapacryl |
| B-193 | eine Verbindung der Formel I | Dicloran |
| B-194 | eine Verbindung der Formel I | Dinobuton |
| B-195 | eine Verbindung der Formel I | Dinocap |
| B-196 | eine Verbindung der Formel I | Nitrothal-isopropyl |
| B-197 | eine Verbindung der Formel I | Tecnazen |
| B-198 | eine Verbindung der Formel I | Fentin-Acetat |
| B-199 | eine Verbindung der Formel I | Fentin-Chlorid |
| B-200 | eine Verbindung der Formel I | Fentin-Hydroxid |
| B-201 | eine Verbindung der Formel I | Isoprothiolane |
| B-202 | eine Verbindung der Formel I | Dithianon |
| B-203 | eine Verbindung der Formel I | Edifenphos |
| B-204 | eine Verbindung der Formel I | Fosetyl |
| B-205 | eine Verbindung der Formel I | Fosetyl-Aluminium |
| B-206 | eine Verbindung der Formel I | Iprobenfos |
| B-207 | eine Verbindung der Formel I | Pyrazophos |
| B-208 | eine Verbindung der Formel I | Tolclofos-methyl |
| B-209 | eine Verbindung der Formel I | Chlorothalonil |
| B-210 | eine Verbindung der Formel I | Dichlofluanid |
| B-211 | eine Verbindung der Formel I | Dichlorophen |
| B-212 | eine Verbindung der Formel I | Flusulfamide |
| B-213 | eine Verbindung der Formel I | Hexachlorbenzene |
| B-214 | eine Verbindung der Formel I | Pencycuron |
| B-215 | eine Verbindung der Formel I | Pentachlorphenol und Salze davon |
| B-216 | eine Verbindung der Formel I | Phthalide |
| B-217 | eine Verbindung der Formel I | Quintozene |
| B-218 | eine Verbindung der Formel I | Thiophanate Methyl |
| B-219 | eine Verbindung der Formel I | Tolylfluanid |
| B-220 | eine Verbindung der Formel I | N-(4-Chlor-2-nitro-phenyl)-N-ethyl-4- |
| B-221 | eine Verbindung der Formel I | methyl-benzolsulfonamid Phosphorige Säure und ihre Salze |
| B-222 | eine Verbindung der Formel I | Schwefel |
| B-223 | eine Verbindung der Formel I | Bordeaux Brühe |
| B-224 | eine Verbindung der Formel I | Kupferacetat |
| B-225 | eine Verbindung der Formel I | Kupferhydroxid |
| B-226 | eine Verbindung der Formel I | Kupferoxychlorid |
| B-227 | eine Verbindung der Formel I | basisches Kupfersulfat |
| B-228 | eine Verbindung der Formel I | Biphenyl |
| B-229 | eine Verbindung der Formel I | Bronopol |
| B-230 | eine Verbindung der Formel I | Cyflufenamid |
| B-231 | eine Verbindung der Formel I | Cymoxanil |
| B-232 | eine Verbindung der Formel I | Diphenylamin |
| B-233 | eine Verbindung der Formel I | Metrafenon |
| B-234 | eine Verbindung der Formel I | Mildiomycin |
| B-235 | eine Verbindung der Formel I | Oxin-Kupfer |
| B-236 | eine Verbindung der Formel I | Prohexadione-Calcium |
| B-237 | eine Verbindung der Formel I | Spiroxamin |
| B-238 | eine Verbindung der Formel I | Tolylfluanid |
| B-239 | eine Verbindung der Formel I | N-(Cyclopropylmethoxyimino-(6-difluormethoxy-2,3-difluor-phenyl)-methyl)-2-phenyl acetamid |
| B-240 | eine Verbindung der Formel I | N'-(4-(4-Chlor-3-trifluormethyl-phenoxy)-2,5-dimethyl-phenyl)-N-ethyl-N-methyl-formamidin |
| B-241 | eine Verbindung der Formel I | N'-(4-(4-Fluor-3-trifluormethyl-phenoxy)-2,5-dimethyl-phenyl)-N-ethyl-N-methyl-formamidin |
| B-242 | eine Verbindung der Formel I | N'-(2-Methyl-5-trifluormethyl-4-(3-trimethyl-silanyl-propoxy)-phenyl)-N-ethyl-N-methylformamidin |
| B-243 | eine Verbindung der Formel I | N'-(5-Difluormethyl-2-methyl-4-(3-tri-methylsilanyl-propoxy)-phenyl)-N-ethyl-N-methylformamidin |

Die voranstehend als Komponente 2 genannten Wirkstoffe II, ihre Herstellung und ihre Wirkung gegen Schadpilze sind allgemein bekannt (vgl.: http://www.hclrss.demon.co.uk/index.html); sie sind kommerziell erhältlich. Die nach IUPAC benannten Verbindungen, ihre Herstellung und ihre fungizide Wirkung sind ebenfalls bekannt [vgl. EP-A 226 917; EP-A 10 28 125; EP-A 10 35 122; EP-A 12 01 648; WO 98/46608; WO 99/24413; WO 03/14103; WO 03/053145; WO 03/066609; WO 04/049804].

### Synthesebeispiele

### 1) Synthese von (4-Fluorbenzoyl)format

Eine auf -65 °C gekühlte Lösung von Diethyloxalat (30.0 g, 205.2 mmol) in einem Gemisch aus Tetrahydrofuran und Diethylether (1:1, 300 ml) wurde tropfenweise mit 4-Fluorphenylmagnesiumbromid (205 ml, 205 mmol, erhalten durch Reaktion von 4-Fluorbrombenzol mit Magnesiumspänen in Tetrahydrofuran). Nach beendeter Zugabe wurde weitere 30 min bei dieser Temperatur gerührt und anschließend auf - 25 °C erwärmt. Nach Zugabe von wässriger Salzsäure (340 ml einer 1 M Lösung) wurde die organische Phase abgetrennt und die wässrige Phase mit Essigester extrahiert (1 x 300 ml). Die vereinigten organischen Phasen wurden mit gesättigter Kochsalzlösung gewaschen (2 x 200 ml), über Natriumsulfat getrocknet, filtriert und vom Lösungsmittel befreit. Nach säulenchromatographischer Aufreinigung (Kieselgel, Hexan/Dichlormethan 3:2) wurde (4-Fluorbenzoyl)format als gelbes Öl (35.0 g, 87 %) erhalten.
H-NMR (300 MHz, CDCl₃): δ 8.10-8.05 (m, 2H), 7.20-7.16 (m, 2H), 4.44 (q, J = 7.0 Hz, 2H), 1.42 (t, J = 7.0 Hz, 3H).

### 2) Synthese von [(2,5-Dichlorthiophen-3-yl)methyl]triphenylphosphoniumchlorid

Zu einer Lösung von 2,5-Dichlor-3-(chlormethyl)thiophen (1.6 g, 8.0 mmol) in Acetontril (40 ml) wurde Triphenylphosphan (2.1 g, 8.0 mmol) zugegeben. Die Reaktionslösung wurde 24 Stunden bei 82°C gerührt, auf Raumtemperatur abgekühlt und das Lösungsmittel abdestilliert. Der erhaltene Rückstand wurde mit Diethylether aufgeschlämmt und das Produkt (2.4 g, 65 %) in Form eines farblosen Feststoffes abfiltriert.

### 3) Synthese von Ethyl 3-(2,5-Dichlorthiophen-3-yl)-2-(4-fluorphenyl)acrylat

Kalium-tert-butanolat (217 mg, 1.9 mmol) wurde zu einer eisgekühlten Lösung von [(2,5-Dichlorthiophen-3-yl)methyl]triphenylphosphoniumchlorid (1.0 g, 2.1 mmol) in Toluol (15 ml) gegeben. Nach 30 min bei dieser Temperatur wurde (4-Fluorbenzoyl)format (444 g, 2.2 mmol) in Toluol tropfenweise zugegeben und nach beendeter Zugabe innerhalb einer Stunde auf Raumtemperatur erwärmt. Anschließend wurde die Reaktionsmischung mit Essigester (20 ml) versetzt und mit gesättigter Kochsalzlösung (2x10 ml) gewaschen. Die organische Phase wurde abgetrennt, über Natriumsulfat getrocknet, filtriert und das Lösungsmittel im Vakuum entfernt. Der erhaltene Rückstand wurde säulenchromatographisch (Kieselgel, Hexan/Essigester 20:1) aufgereinigt. Nach Vereinigen der entsprechenden Fraktionen wurde Ethyl 3-(2,5-Dichlorthiophen-3-yl)-2-(4-fluorphenyl)acrylat (290 mg, 37%) als 2:1 Mischung von trans zu cis-Isomer in Form eines farblosen Öles erhalten.
H-NMR (300 MHz, CDC13): δ 7.76 (s, 1H), 7.19-7.09 (m, 4H), 5.79 (s, 1H), 4.26 (q, J = 7.0 Hz, 2H), 1.29 (t, J = 7.0 Hz, 3H).

### 4) Synthese von (E)-3-(2,5-Dichlorthiophen-3-yl)-2-(4-fluorphenyl)prop-2-en-1-ol

Zu einer Lösung von Ethyl 3-(2,5-Dichlorthiophen-3-yl)-2-(4-fluorphenyl)acrylat (280 mg, 0.8 mmol) in wasserfreiem Tetrahydrofuran (50 ml) wurde bei -78 °C Diisobutylaluminiumhydrid (4.4 ml einer 1 M Lösung in Toluol, 4.4 mmol) tropfenweise zugegeben. Die Reaktionslösung wurde zwischen -78 und -60°C für 25 min gerührt, bevor bei -60°C Methanol zugegeben wurde. Der entstehende Niederschlag wurde abfiltriert, mit Methanol (10 ml) gewaschen und die vereinigten Filtrate vom Lösungsmittel befreit. Nach säulenchromatographischer Aufreinigung (Kieselgel, Dichlormethan) und Vereinigen der entsprechenden Fraktionen wurde (E)-3-(2,5-Dichlorthiophen-3-yl)-2-(4-fluorphenyl)prop-2-en-1-ol als farbloses Öl (80 mg, 33 %) H NMR (300 MHz, CDCl₃): δ 7.84 (s, 1H), 7.21-7.17 (m, 2H), 7.10-7.06 (m, 2H), 6.61 (s, 1H), 5. 89 (s, 1H), 4.43 (d, J = 8.0 Hz, 2H).

### 5) Synthese von (E)-3-(2,5-Dichlorthiophen-3-yl)-2-(4-fluorphenyl)acrylaldehyd

Eine Lösung von (E)-3-(2,5-Dichlorthiophen-3-yl)-2-(4-fluorphenyl)acrylaldehyd (80 mg, 0.2 mmol) in wasserfreiem Dichlormethan (5 ml) wurde bei 0 °C mit Dess-Martin-Periodinan (122 mg, 0.2 mmol) versetzt. Innerhalb einer Stunde wurde das Reaktionsgemisch auf Raumtemperatur erwärmt, mit Essigester (10 ml) verdünnt und mit gesättigter Natriumhydrogencarbonat-Lösung (2x10 ml) gewaschen. Die organische Phase wurde mit gesättigter Kochsalzlösung behandelt, über Natriumsulfat getrocknet, filtriert und konzentriert. Der Rückstand wurde säulenchromatographisch (Kieselgel, Hexan/Essigester 10:1) aufgereinigt. Der Aldehyd wurde nach Vereinigen der entsprechenden Fraktionen und Entfernen des Lösungsmittels (60 mg, 76 %) erhalten. H-NMR (300 MHz, CDCl₃): δ 9.76 (s, 1H), 7.36 (s, 1H), 7.16 (s, 2H), 7.14 (s, 2H), 6.06 (s, 1H).

### 6) Synthese von [(2SR,3RS)-3-(2,5-Dichlorthiophen-3-yl)-2-(4-fluorphenyl)oxiran-2-yl)methanol

(E)-3-(2,5-Dichlorthiophen-3-yl)-2-(4-fluorphenyl)acrylaldehyd (60 mg, 0.1 mmol) wurde in Methanol (2 ml) gelöst und bei Raumtemperatur mit wässriger NatriumhydroxidLösung (0.01 mmol) und Wasserstoffperoxid (8 mg einer 50 %igen wässrigen Lösung, 0.2 mmol) versetzt. Nach einer Stunde bei dieser Temperatur wurde die Reaktionsmischung auf 0 °C abgekühlt, mit Natriumborhydrid (7 mg, 0.1 mmol) versetzt und nach Auftauen auf Raumtemperatur für eine weitere Stunde nachgerührt. Anschließend wurde die Reaktion durch Zugabe von gesättigter wässriger Ammoniumchlorid-Lösung (2 ml) beendet. Nach Extraktion mit Essigester (20 ml) wurde die organische Phase mit gesättigter Kochsalzlösung (2 x 5 ml) gewaschen, über Natriumsulfat getrocknet, filtriert und vom Lösungsmittel befreit. Der erhaltene Rückstande (56 mg) wurde ohne weitere Aufreinigung im nächsten Reaktionsschritt eingesetzt.
H-NMR (300 MHz, CDCl₃): δ 7.53-7.48 (m, 2H), 7.13-7.07 (m, 2H), 6.82 (s, 1H), 4.05 (d, J = 12.9 Hz, 1H), 3.89 (s, 1H), 3.68 (d, J = 12.9 Hz, 1H).

### 7) Synthese von [(2SR,3RS)-3-(2,5-Dichlorthiophen-3-yl)-2-(4-fluorphenyl)oxiran-2-yl]methyl methansulfonat

Eine eisgekühlte Lösung von [(2SR,3RS)-3-(2,5-Dichlorthiophen-3-yl)-2-(4-fluorphenyl)oxiran-2-yl)methanol (56 mg, 0.1 mmol) in Dichlormethan (4 ml) wurde mit Triethylamin (35 mg, 0.2 mmol) und Methansulfonylchlorid (30 mg, 0.2 mmol) versetzt. Die Reaktionsmischung wurde auf Raumtemperatur erwärmt und für weitere 18 Stun-den gerührt, bevor Essigester zugegeben und mit gesättigter Natriumhydrogencarbonat-Lösung (2 x 5 ml) gewaschen wurde. Die organische Phase wurde über Natriumsulfat getrocknet und das Lösungsmittel im Vakuum entfernt. Der erhaltene Rückstand wurde säulenchromatographisch (Kieselgel, Hexan/Essigester 20:1) aufgereinigt. Man erhielt nach Vereinigen der entsprechenden Fraktionen [(2SR,3RS)-3-(2,5-Dichlorthiophen-3-yl)-2-(4-fluorphenyl)oxiran-2-yl]methyl methansulfonat (30 mg, 43 %) in Form eines farblosen Öles.
H-NMR (300 MHz, CDCl₃): δ 7.50-7.45 (m, 2H), 7.15-7.09 (m, 2H), 6.85 (s, 1H), 4.68 (d, J = 12.0 Hz, 1H), 4.21 (d, J = 12.0 Hz, 1H), 3.94 (s, 1H), 2.80 (s, 3H).

### 8) Synthese von 1-{[(2SR,3RS)-3-(2,5-Dichlorthiophen-3-yl)-2-(4-fluorphenyl)oxiran-2-yl]methyl}-1H-1,2,4-triazol

Eine Mischung von 1,2,4-Triazol (15 mg, 0.2 mmol) und Natriumhydrid (5 mg, 0.2 mmol) in wasserfreiem N,N-Dimethylformamid (1 ml) wurde für 15 min bei Raumtemperatur gerührt. Eine Lösung von [(2SR,3RS)-3-(2,5-Dichlorthiophen-3-yl)-2-(4-fluorphenyl)oxiran-2-yl]methyl methansulfonat (30 mg, 0.05 mmol) in N,N-Dimethylformamid (2 ml) wurde zugegeben und die Reaktionslösung bei Raumtemperatur für fünf Stunden gerührt. Anschließend wurde mit Essigester verdünnt und mit gesättigter Kochsalzlösung (2 x 5 ml) gewaschen. Die organische Phase wurde über Natriumsulfat getrocknet, filtriert und das Lösungsmittel im Vakuum entfernt. Nach säulenchromatographischer Aufreinigung (Kieselgel, Hexan/Essigester 1:1) wurde die Zielverbindung (19 mg, 70 %) erhalten.
H-NMR (300 MHz, CDCl₃): δ 7.88 (s, 1H), 7.82 (s, 1H), 7.31-7.27 (m, 2H), 7.05-6.99 (m, 2H), 6. 91 (s, 1H), 4.80 (d, J = 15.5 Hz, 1H), 4.17 (d, J = 15.5 Hz, 1H), 4.00 (s, 1H).

### Biologischer Teil

### Gewächshaus

### Wirkstoffaufbereitung

Die Wirkstoffe wurden getrennt oder gemeinsam als eine Stammlösung aufbereitet mit 25 mg Wirkstoff, welcher mit einem Gemisch aus Aceton und/oder DMSO und dem Emulgator Wettol EM 31 (Netzmittel mit Emulgier- und Dispergierwirkung auf der Basis ethoxylierter Alkylphenole) im Volumen-Verhältnis Lösungsmittel-Emulgator von 99 zu 1 ad 10 ml aufgefüllt wurde. Anschließend wurde ad 100 ml mit Wasser aufgefüllt. Diese Stammlösung wurde mit dem beschriebenen Lösungsmittel-Emulgator-Wasser Gemisch zu der unten angegeben Wirkstoffkonzentration verdünnt.

### Anwendungsbeispiel 1 - Protektive Wirksamkeit gegen Puccinia recondita an Weizen (Weizenbraunrost) (Puccrt P1)

Blätter von in Töpfen gewachsenen Weizensämlingen wurden mit einer wässriger Suspension in der unten angegebenen Wirkstoffkonzentration bis zur Tropfnässe besprüht. Am nächsten Tag wurden die behandelten Pflanzen mit einer Sporensuspension des Weizenbraunrostes (Puccinia recondita) inokuliert. Anschließend wurden die Pflanzen für 24 Stunden in eine Kammer mit hoher Luftfeuchtigkeit (90 bis 95 %) bei 20 bis 22 ° C gestellt. Während dieser Zeit keimten die Sporen aus und die Keimschläuche drangen in das Blattgewebe ein. Am folgenden Tag wurden die Versuchspflanzen ins Gewächshaus zurückgestellt und bei Temperaturen zwischen 20 und 22 ° C und 65 bis 70 % relativer Luftfeuchte für weitere 7 Tage kultiviert. Dann wurde das Ausmaß der Rostpilzentwicklung auf den Blättern visuell ermittelt.

Die mit dem Wirkstoff 1-{[(2SR,3RS)-3-(2,5-Dichlorthiophen-3-yl)-2-(4-fluorphenyl)oxiran-2-yl]methyl}-1H-1,2,4-triazol mit 250 ppm - haltiger wässriger Wirkstoffaufbereitung behandelten Pflanzen zeigten einen Befall von 0%, während die unbehandelten Pflanzen zu 90% befallen waren.

### Anwendungsbeispiel 2 - Wirksamkeit gegen die Netzfleckenkrankheit der Gerste verursacht durch Pyrenophora teres bei 1 Tag protektiver Anwendung (Pyrnte P1)

Blätter von in Töpfen gewachsenen Gerstenkeimlingen wurden mit wässriger Suspension in der unten angegebenen Wirkstoffkonzentration bis zur Tropfnässe besprüht. 24 Stunden nach dem Antrocknen des Spritzbelages wurden die Versuchspflanzen mit einer wässrigen Sporensuspension von Pyrenophora [syn. Drechslera] teres, dem Erreger der Netzfleckenkrankheit inokuliert. Anschließend wurden die Versuchspflanzen im Gewächshaus bei Temperaturen zwischen 20 und 24° C und 95 bis 100 % relativer Luftfeuchtigkeit aufgestellt. Nach 6 Tagen wurde das Ausmaß der Krankheitsentwicklung visuell in % Befall der gesamten Blattfläche ermittelt.

Die mit dem Wirkstoff 1-{[(2SR,3RS)-3-(2,5-Dichlorthiophen-3-yl)-2-(4-fluorphenyl)oxiran-2-yl]methyl}-1H-1,2,4-triazol mit 250 ppm - haltiger wässriger Wirkstoffaufbereitung behandelten Pflanzen zeigten einen Befall von 15%, während die unbehandelten Pflanzen zu 90% befallen waren.

## Patentansprüche

1. Azolylmethyloxirane der allgemeinen Formel I 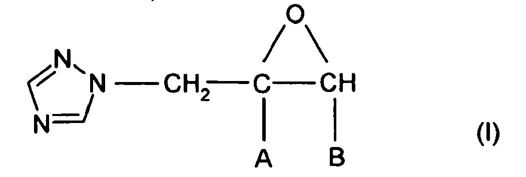 worin
A oder B für Thienyl, das durch einen bis drei der folgenden Substituenten Ha- logen, C₁-C₄-Alkyl, C₁-C₄-Alkoxy, C₁-C₄-Halogenalkyl oder C₁-C₄- Halogenalkoxy substituiert ist,
und der jeweils andere Substituent
A oder B für Phenyl steht, das durch ein bis drei der folgenden Substituenten Halogen, C₁-C₄-Alkyl, C₁-C₄-Alkoxy, C₁-C₄-Halogenalkyl oder C₁-C₄- Halogenalkoxysubstituiert ist,
sowie deren für Pflanzen verträgliche Säureadditions- oder Metallsalze.

2. Verbindungen nach Anspruch 1, bei denen das 5-gliedrige Heteroaryl durch ein bis drei der folgenden Substituenten Halogen, C₁-C₄-Alkyl oder C₁-C₄-Alkoxy, substituiert ist.

3. Verbindungen nach Anspruch 1 oder 2, bei dem das Phenyl durch Halogen, C₁-C₄-Alkyl oder C₁-C₄-Alkoxy, substituiert ist.

4. Verwendung von Verbindungen der Formel I gemäß einem der Ansprüche 1 bis 3 und ihrer Säureadditions- oder Metallsalze zur Bekämpfung von pflanzenpathogenen Pilzen.

5. Mittel für den Pflanzenschutz, enthaltend einen festen oder flüssigen Träger und eine Verbindung der Formel I gemäß einem der Ansprüche 1 bis 3 und/oder ein Säureadditions- oder Metallsalz davon.

6. Saatgut, enthaltend wenigstens eine Verbindung der Formel I gemäß einem der Ansprüche 1 bis 3 und/oder ein Säureadditions- oder Metallsalz davon.

7. Verfahren zur Bekämpfung von pflanzenpathogenen Pilzen, **dadurch gekennzeichnet, dass** man die Pilze, oder die vor Pilzbefall zu schützenden Materialien, Pflanzen, den Boden oder Saatgüter mit einer wirksamen Menge einer Verbindung der Formel I gemäß einem der Ansprüche 1 bis 3 oder einem Säureadditions- oder Metallsalz davon behandelt.

## Claims

1. An azolylmethyloxirane of the general formula I 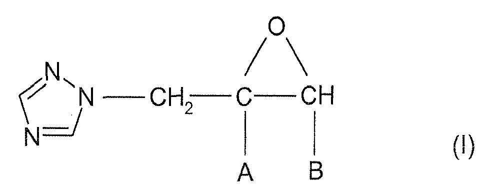 in which
A or B is thienyl which is substituted by one to three of the following substituents: halogen, C₁-C₄-alkyl, C₁-C₄-alkoxy, C₁-C₄- haloalkyl or C₁-C₄-haloalkoxy,
and the respective other substituent
A or B is phenyl which is substituted by one to three of the following substituents: halogen, C₁-C₄-alkyl, C₁-C₄-alkoxy, C₁-C₄- haloalkyl or C₁-C₄-haloalkoxy,
or a plant-compatible acid addition salt or metal salt thereof.

2. The compound according to claim 1 in which the 5- membered heteroaryl is substituted by one to three of the following substituents: halogen, C₁-C₄-alkyl or C₁-C₄-alkoxy.

3. The compound according to claim 1 or 2 in which the phenyl is substituted by halogen, C₁-C₄-alkyl or C₁-C₄-alkoxy.

4. The use of a compound of the formula I according to any of claims 1 to 3 or an acid addition salt or metal salt thereof for controlling phytopathogenic fungi.

5. A crop protection composition comprising a solid or liquid carrier and a compound of the formula I according to any of claims 1 to 3 and/or an acid addition salt or metal salt thereof.

6. Seed comprising at least one compound of the formula I according to any of claims 1 to 3 and/or an acid addition salt or metal salt thereof.

7. A method for controlling phytopathogenic fungi wherein the fungi or the materials, plants, the soil or seed to be protected against fungal attack are/is treated with an effective amount of a compound of the formula I according to any of claims 1 to 3 or an acid addition salt or metal salt thereof.

## Revendications

1. Azolylméthyloxirannes de formule générale I 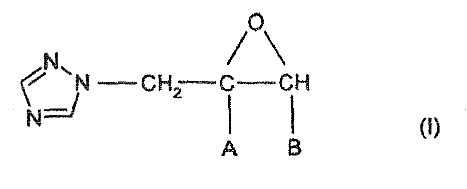 dans laquelle
A ou B représente un radical thiényle, qui est substitué par un à trois des substituants suivants halogène, alkyle en C₁-C₄, alcoxy en C₁-C₄, halogénoalkyle en C₁-C₄ ou halogénoalcoxy en C₁-C₄,
et l'autre substituant, respectivement
A ou B représente un radical phényle, qui est substitué par un à trois des substituants suivants halogène, alkyle en C₁-C₄, alcoxy en C₁-C₄, halogénoalkyle en C₁-C₄ ou halogénoalcoxy en C₁-C₄,
ainsi que leurs sels métalliques ou d'addition avec des acides acceptables pour des plantes.

2. Composés selon la revendication 1, dans lesquels le radical hétéroaryle à 5 chaînons est substitué par un à trois des substituants suivants halogène, alkyle en C₁-C₄ ou alcoxy en C₁-C₄.

3. Composés selon la revendication 1 ou 2, dans lesquels le radical phényle est substitué par halogène, alkyle en C₁-C₄ ou alcoxy en C₁-C₄.

4. Utilisation de composés de formule I selon l'une quelconque des revendications 1 à 3 et de leurs sels métalliques ou d'addition avec des acides, pour la lutte contre des champignons phytopathogènes.

5. Composition pour la protection des plantes, contenant un support liquide ou solide et un composé de formule I selon l'une quelconque des revendications 1 à 3 et/ou un sel métallique ou d'addition avec un acide d' un tel composé.

6. Semence, contenant un composé de formule I selon l'une quelconque des revendications 1 à 3 et/ou un sel métallique ou d'addition avec un acide d'un tel composé.

7. Procédé pour la lutte contre des champignons phytopathogènes, **caractérisé en ce qu'**on traite les champignons ou les matériaux, les plantes, les sols ou les semences à protéger contre l'attaque des champignons, par une quantité efficace d'un composé de formule I selon l'une quelconque des revendications 1 à 3 ou d'un sel métallique ou d'addition avec un acide d' un tel composé.
